# EUROPEAN PATENT APPLICATION

(11) **EP 2 031 072 A1**
(43) Date of publication of application: **04.03.2009**
(21) Application number: 07115056.9
(22) Date of filing: 27.08.2007
(51) Int. Cl.: C12Q 1/68

(54) **Method for in-vitro diagnosing a predisposition to smoking and a pharmaceutical composition for treating tobacco abuse**

(71) Applicant: Ziegler, Andreas, 14050 Berlin (DE)
(72) Inventor: Ziegler, Andreas, 14050 Berlin (DE); Uchanska-Ziegler, Barbara, 14050 Berlin (DE)
(74) Representative: Ziebig, Marlene

(57) **Abstract**

The invention relates to a method for in-vitro diagnosing a predisposition to smoking by incubating a sample of a body fluid taken from a human with substances being specific to at least one single nucleotide polymorphism (SNP) that is selected from the group represented by tag-SNP rs3130845, or at least one single amino acid polymorphism (SAAP) that is encoded thereby, and detecting the specific incubation products. Another object of the invention concerns a pharmaceutical composition for prophylaxis or therapy of tobacco abuse. The invention also relates to a method for screening substances with the property to reduce the predilection for ingredients of tobacco mixtures or tobacco smoke.

## Description

The invention relates to a method for in-vitro diagnosing a predisposition to smoking by incubating a sample of a body fluid taken from a human with substances being specific to at least one single nucleotide polymorphism (SNP) that is selected from the group represented by tag-SNP rs3130845, or at least one single amino acid polymorphism (SAAP) that is encoded by any of said SNPs, and detecting the specific incubation products. Another object of the invention concerns a pharmaceutical composition for prophylaxis or therapy of tobacco abuse. The invention also relates to a method for screening substances with the property to reduce the predilection for ingredients of tobacco mixtures or tobacco smoke.

Smoking is a highly complex human behavior that affects an increasing number of adolescents. There are currently about 1.3 billion smokers worldwide, and their number is expected to rise to 1.7 billion by the year 2025. Smoking is associated with many serious health problems, including cancer of various organs, coronary artery disease, as well as several autoimmune disorders and must thus be considered as a leading cause of death and disability worldwide. Although there is general agreement that nicotine is the core addictive component of cigarette smoke, there are hundreds of further substances that may influence the initiation and continuation of tobacco abuse.

It is known that the dependence on smoking is modulated by genetic factors apart from social and psychological factors. First twin studies have proven a relative risk between 46 % and 84 %. There are several genes, which are associated to smoking behavior. In particular, cytochrome P450 enzymes play an important role in the detoxication metabolism, whereas dopamine and the dopamine receptor enhance the impact of nicotine. It was recently shown by Füst et al. (Int. Immunol. 16, 1507-1514, 2004) that a haplotype of the human major histocompatibility (HLA) complex is associated with smoking behavior. Individuals with this most common HLA haplotype among Europeans, which is termed HLA-A1-B8-DR3, have a five times higher chance of being smokers than individuals without this haplotype.

Miretti et al. (Am. J. Hum. Genet. 76, 634-646, 2005) have previously found that strong linkage disequilibrium extends to the extended HLA complex (xHLA). Therefore, genes within the xHLA may contribute to the association between smoking and the HLA-A1-B8-DR3 haplotype as well, with a clear-cut gender bias towards females (Füst et al., Int. Immunol. 16, 1507-1514, 2004). However, prior art lacks a definitive and unbiased association between smoking and, hence, smoking-associated diseases, to specific genes within the xHLA. This is to a large extent due to the exceptionally strong linkage disequilibrium that is a hallmark of the HLA-A1-B8-DR3 haplotype. It causes a non-random association of alleles from coupled gene loci deviating from the frequency expected by the Hardy-Weinberg-equilibrium in the population.

Therefore, the technical problem forming the basis of the present invention is to provide a method for diagnosing a predisposition to smoking, which makes a simple and fast determination of an elevated risk for initiating or continuing tobacco consumption possible. It is another problem of the invention to provide a pharmaceutical composition allowing an effective application in prevention and therapy of tobacco abuse. It is still another problem to provide a method for screening substances that effectively reduce the predilection for ingredients of tobacco smoke.

The present invention solves the problem by providing a method for in-vitro diagnosing a predisposition to smoking comprising the steps of incubating a sample of a body fluid taken from a human with substances being specific to at least one SNP that is selected from the group comprising the SNPs rs6908726, rs9393929, rs6456835, rs1233610, rs1233619, rs1742753, rs3131343, rs4324798, rs3118357, rs9257248, rs3131335, rs3132388, rs3132389, rs3131336, rs3118370, rs3131093, rs3132392, rs3135309, rs3130838, rs3135293, rs2230683, rs3118361, rs3130895, rs3131073, rs3130845, rs3130837, rs3129791, rs3130891, rs3130893, rs3131085, rs3129173, rs3117326, rs3117425 and rs3749971, or at least one single amino acid polymorphism (SAAP) that is encoded by any of said SNPs, and detecting the specific incubation products.

It has been surprisingly demonstrated by the inventors that only the aforementioned group of SNPs is correlated with smoking behavior. The correlation between the identified SNPs and smoking is even of higher significance than the association between HLA-A1-B8-DR3 and this behavioral trait. The SNPs are selected from the group represented by tag-SNP #51, a fact that is clearly to be seen in Tables 1, 2 and 3. Additional, tag-SNPs in general are described by Miretti et al. (Am. J. Hum. Genet. 76, 634-646, 2005), which is incorporated as reference in the disclosure of the invention hereby. The tag-SNP #51 is actually represented by rs3130845, which is a member and eponym by chance of this group. All SNPs are
characterized by a strong linkage disequilibrium among each other, which is almost considered to be absolute. The tag-SNP rs3130845 is passed on along with the other 33 SNPs in almost each recombination event in the population. That means, the typing of one SNP within this group results in typing the other 33 SNP with the utmost probability. Consequently, reference to "a SNP" includes a specified SNP, several distinct SNPs or even any SNP represented by the tag-SNP rs3130845. Reference to a specified SNP shall not be understood to limit the scope of protection since the SNP members of the group represented by the tag-SNP rs3130845 may be replaced by each other. The teaching of the present specification concerning a specific SNP, such as rs3749971, is considered as valid and applicable without restrictions to other members of the group represented by the tag-SNP rs3130845 if these SNPs are expedient therein. It is also not excluded that further SNPs will be characterized, which also exhibit a strong linkage disequilibrium to some or even all members of the group represented by tag-SNP rs3130845. Therefore, the teaching of the present invention is not restricted to the currently known 34 SNPs, but shall cover each SNP of strong linkage disequilibrium to them.

The term "linkage disequilibrium" denotes a non-random distribution of loci, such as SNP, resulting in a more frequently combined appearance as expected. It is observed if loci are closely located on a chromosome causing an allele appearance dependent on each other. As used herein, the terms "almost absolute linkage disequilibrium" or "with the utmost probability" refer to a combined heredity transmission in 95% of events, i.e. in at least 171 of 180 chromosomes.

The term "encoded" refers to the fact that the SNP-based nucleotide exchange may result in an amino acid exchange depending on the position within a triplet in the reading frame and/or the degeneration of the genetic code in respect of a certain amino acid.

In a preferred embodiment of the inventive method for diagnosis, the SNP rs3749971 within the OR12D3 gene or the SAAP specifying isoleucine at position 97 within the OR12D3 protein is selected. rs3749971 is the dbSNP assigned reference SNP ID by NCBI (http:/www.ncbi.nlm.nih.gov) and generally accepted. Nevertheless, the SNP rs3749971 may be named in another way, such as ss12677271, ss24339648 or ss48423304. The same is valid for other SNP members of the group represented by tag-SNP rs3130845 and tag-SNP rs3130845 itself. OR12D3 is the approved term of HUGO, the Human Genome Organization, http://www.hugo-international.org. Furthermore, the OR12D3 gene has several synonyms, such as OR6-27, hs6M1-27, bA150A6.1 or HsOR6.3.18.

The invertors surprisingly found that only the SNP rs3749971, which is part of the xHLA, represents a coding, non-synonymous SNP and is correlated with smoking behavior. This SNP is located within the gene OR12D3, a polymorphic member of the odorant receptor (OR) gene clusters within the telomeric xHLA. With about 400 potentially functional members, the family of olfactory-receptor genes provides the basis for odor perception. In detail, the rs3749971 polymorphism leads to an exchange of threonine by isoleucine at position 97 that affects a putative ligand-binding region of the OR12D3 protein. Indeed, this non-synonymous SNP is very strongly associated with HLA-A1-B8-DR3, but not with other HLA haplotypes as the majority of the rs3749971^{T} carriers but only a minority of the non-carriers were found to be HLA-A1-B8-DR3 positive. A majority amounts to at least 50 %, more preferably at least 70 %, whereas a minority amounts to less than 20 %, more preferably less than 5 %. The polymorphism influences the HLA haplotype-dependent differential recognition of cigarette smoke components, thereby predisposing individuals with rs3749971^{T} or further SNPs that are represented by the tag-SNP rs3130845, to tobacco abuse.

The linkage of smoking to the distinct odorant receptor allele is utilized for the in-vitro diagnosis of the genetic predisposition to smoking by means of a single marker. That means, the inventive principle underlying the present diagnostic method comprises prospecting for a polymorphism which can be detected either on the genetic level as represented by tag-SNP rs31308545, preferably SNP rs3749971^{T}, or on the protein level as represented by the translation product including the matching SAAP. It is to be understood that gene products of the rs3749971^{T} allele also represent designated targets in the diagnostic method of the invention. The term "gene product" denotes molecules that are formed from the substrate of rs3749971^{T} allele by biochemical, chemical or physical reactions, such as DNA synthesis, transcription, splicing, fragmentation or methylation. Preferred gene products of the invention are amplified dsDNA, or cDNA or mRNA.

In a first step, a sample of a body fluid is taken from a human to be tested. The withdrawal of the body fluid sample follows good medical practice. In the present invention, the sample of body fluid preferably consists of blood, serum, plasma, saliva or urine, more preferably saliva. The sample may be purified to remove disturbing substances, such as inhibitors for the formation of hydrogen bonds. Alternatively, the genetic material or the OR12D3 protein, respectively, can be concentrated in the sample. Downstream processing and/or concentrating are preferably performed by the methods of precipitation, dialysis, gel filtration, gel elution or chromatography, such as HPLC or ion exchange chromatography. It is recommended to combine several methods for better yields.

The sample of the body fluid taken from the human is incubated with substances that are specific for the SNP rs3749971^{T} within the OR12D3 gene, and/or further SNPs that are represented by the tag-SNP rs31308545, or the SAAP specifying isoleucine at position 97 within the OR12D3 protein. The sequence of the modified OR12D3^{97Ile} protein is given in the sequence listing according to the amino acid sequence of SEQ ID NO: 4.

The term "specific substances" as used herein comprises molecules with high affinity to at least one SNP selected from the group represented by tag-SNP rs3130845 and the flanking regions of these respective SNP, the threonine to isoleucine polymorphism at position 97 of the OR12D3 protein (SEQ ID NO: 4) and its flanking regions, or variants of the flanking regions in order to ensure a reliable binding. The degree of variation between the flanking regions and its variants is inevitably limited by the requirement of SNP or SAAP recognition within the structural context by means of the specific substances. Preferably, the homology amounts to at least 85 %. Possible alterations comprise deletion, insertion, substitution, modification and addition of at least one nucleotide or amino acid, respectively, or the fusion with another nucleic acid or protein.

The recognition of the target SNP or SAAP according to the invention can be realized by a specific interaction with substances on the primary, secondary and/or tertiary structure level of a nucleic acid sequence bearing rs3749971^{T} or an amino acid sequence expressing the SAAP in the matching reading frame of the OR12D3 protein. The coding function of genetic information favors the primary structure recognition, i.e. the recognition of the nucleotide sequence of the rs3749971^{T} allele or at least a difference in pattern at the site of genetic polymorphism. Contrary to that, the three-dimensional structure is mainly to be considered for SAAP recognition within the OR12D3 protein. The exchange of the hydrophilic amino acid threonine by isoleucine, a residue with a hydrophobic side chain, at position 97, is expected to alter the physicochemical properties of the OR12D3 protein. Models locate the isoleucine residue at the end of the first extracellular loop or at the beginning of the third transmembrane region, close to the ligand-binding site. Consequently, the threonine to isoleucine exchange at position 97 comes along with a conformational change of the receptor that exerts an influence on its ligand specificity. Both, the design of ligands fitting into the binding pocket or substances targeting the conformation anywhere may represent suitable approaches for the recognition of the protein of SEQ ID NO: 4 or variants thereof.

In the context of the present invention, the term "recognition" - without being limited thereto - relates to any type of interaction between the specific substances and the target, particularly covalent or non-covalent binding or association, such as a covalent bond, hydrophobic/hydrophilic interactions, van der Waals forces, ion pairs, hydrogen bonds, ligand-receptor interactions, interactions between epitope and antibody binding site, nucleotide base pairing, and the like. Such association may also encompass the presence of other molecules such as peptides, proteins or other nucleotide sequences.

The specific substances of the invention comprise nucleic acids, peptides, proteins, carbohydrates, polymers and small molecules having a molecular weight between 50 and 1.000 Da. The specific substances express a sufficient sensitivity and specificity in order to ensure a reliable detection.

The proteins or peptides are preferably selected from the group consisting of antibodies, cytokines, lipocalins, receptors, lectins, avidins, lipoproteins, glycoproteins, oligopeptides, peptide ligands and peptide hormones. More preferably, antibodies are used as specific substance to interact with the modified OR12D3 protein of SEQ ID NO: 4. "Antibody" denotes a polypeptide essentially encoded by an immunoglobulin gene or fragments thereof. According to the invention, antibodies are present as intact immunoglobulins or a number of well-characterized fragments. Fragments are preferably selected from the group consisting of F_{ab} fragments, F_{c} fragments, single chain antibodies (scFv), variable regions, constant regions, H chain (V_{H}), and L chain (V_{L}), more preferably F_{ab} fragments and scFv. Fragments, such as F_{ab} fragments and F_{c} fragments, can be produced by cleavage using various peptidases. Furthermore, fragments can be engineered and recombinantly expressed, preferably scFv.

The term "nucleic acid" refers to a natural or synthetic polymer of single- or double-stranded DNA or RNA alternatively including synthetic, non-natural or modified nucleotides, which can be incorporated in DNA or RNA polymers. Each nucleotide consists of a sugar moiety, a phosphate moiety, and either a purine or pyrimidine residue. The nucleic acids are preferably single or double stranded DNA or RNA, primers, antisense oligonucleotides, ribozymes, DNA enzymes, aptamers and/or siRNA, or parts thereof. The nucleic acids can be optionally modified as phosphorothioate DNA, locked nucleic acid (LNA), peptide nucleic acid (PNA) or spiegelmer.

Particular preferred nucleic acids to be used as SNP-specific substances are primers. Examples of such primers may include at least a pair of primers constituted of a first primer, which has five or more continuous nucleotides of a first nucleotide sequence and a second primer, which has five or more continuous nucleotides of a second nucleotide sequence, whereby the first and the second nucleotide sequences differ in at least one nucleotide. It is a particular preferred embodiment of the invention that the forward primer comprises the DNA sequence 5' AGCGAAGAGGATTGCAGATGGC 3' (SEQ ID NO: 1) and the reverse primer comprises the DNA sequence 5' ATACAGCCTATATCTTTTCTAGGCTGTATCAT 3' (SEQ ID NO: 3). Simultaneously or sequentially, the reverse primer sequence 5' ATACAGCCTATATCTTTTCTAGGCTGTATCAC 3' (SEQ ID NO: 2) can be applied as control, especially in the experimental frame of genotyping by polymerase chain reaction (PCR).

The specific substances of the invention are incubated with the sample of body fluid for a distinct time that depends on the kind of substance and/or target. As used herein, "incubation" denotes the contacting of specific substances with the OR12D3^{97Ile} protein or the SNP allele of interest, such as the rs3749971^{T} allele, which can be realized without a chemical conversion, e.g. by an aptamer- OR12D3^{97Ile} complex, or may involve a biochemical reaction, e.g. by an enzyme- rs3749971^{T} complex. Adding chemical solutions and/or applying physical procedures, e.g. impact of heat, may improve the accessibility of the SNP in the sample. In a preferred embodiment of the present invention, the incubating step comprises the hybridization of primers for the rs3749971^{T} allele and the amplification of the region between them. The primers are designed based on the nucleotide sequence information of the region flanking the site of genetic polymorphism. The primers may be designed so as to amplify a region of 100 to 200 bp in length. The nucleic acid amplification method includes, but is not particularly limited to, a PCR, preferably a real-time PCR.

The specific substances can be labeled, in doing so the labeling depends on their inherent features and the detection method to be applied. For the detection of the specific incubation products, the applied methods depend on the specific incubation products to be monitored and are well known to the skilled artisan. Examples of suitable detection methods according to the present invention are luminescence, VIS coloring, radioactive emission, electrochemical processes or mass spectrometry.

In an embodiment of the invention, the incubation products are detected by the labeling of the nucleic acids, preferably the nucleic acids acting as specific substances. A labeling method is not particularly limited as long as a label is easily detected. In a preferred embodiment of the present invention, the nucleic acids are labeled with digoxigenin, biotin, chemiluminescence substances, fluorescence dyes well known in the art or radioactive isotopes. Preferred isotopes for labeling nucleic acids in the scope of the invention are ³H, ¹⁴C, ³²P, ³³P, ³⁵S, or ¹²⁵I, more preferred ³²P, ³³P, or ¹²⁵I.

After performing an amplification reaction of a nucleic acid using the biological sample to be analyzed and primers as described above, it is checked whether the nucleic acid is amplified or not. In order to facilitate the detection of an amplified nucleic acid bearing rs3749971^{T}, a primer may be labeled in advance. Examples of applicable fluorescent labels include FAM™, TET™, HEX™, TAMRA™ and ROX™ manufactured by Applied Biosystems. In these cases, either the 5' end or the 3' end of a primer may be labeled, preferably the 5' end. Alternatively, the nucleic acid may be labeled during PCR by using labeled nucleotides, or even after PCR is completed. Light emission is measured by a general-purpose luminescence determination device.

In particular, a nucleic acid microarray may be applied. This assay uses two sets of primers having the same nucleotide sequence but fluorescence labels of different wavelength. The amplification of one distinct expression of polymorphism is performed by one set of primers, such as the primer sequences of SEQ ID NOs: 1 and 2, whereas the amplification of another expression type at the site of genetic polymorphism is performed by the second set of primers, such as the sequences of SEQ ID NOs: 1 and 3. Since the primer composition and length of the amplification product set the time period and temperature of the denaturation, annealing and/or elongation step, the amplification reactions are particularly run in parallel tubes, and subsequently the amplified nucleic acid fragments are mixed and the fluorescence is detected by the nucleic acid microarray. If using the preferred primer sequence of SEQ ID NOs: 1 to 3, however, the amplification can be simultaneously performed in a single trial, assessing the genotype of many different individuals.

When mass spectroscopy is used, a primer may be designed so as to allow the amplification of nucleic acid fragments having a length that varies with the pattern of the site of polymorphism.

The presence or absence of an amplified nucleic acid fragment can also be checked by subjecting a reaction solution to electrophoresis, such as for single-strand conformation polymorphism (SSCP) analysis, which may be performed by capillary electrophoresis. However, other electrophoresis methods, for instance gel electrophoresis, are also applicable and well known to those skilled in the art.

The detection of specific incubation products identifies individuals with an elevated risk for initiating or continuing tobacco consumption. In detail, the detection of incubation products amounts to an elevated risk for initiating or continuing smoking of at least the 10-fold, preferably at least the 15-fold, more preferably at least the 20-fold. Furthermore, the presence of specific incubation products indicates a predisposition for acquiring disorders, such as lung cancer or Graves' disease, that are clearly connected with smoking. Since the HLA-A1-B8-DR3 haplotype predisposes to smoking, and is also associated with various autoimmune diseases some of which correlate with this behavioral trait, these associations must also extend to the A1-B8-DR3-associated rs3749971^{T} allele. Although strong linkage disequilibrium between HLA-A1-B8-DR3 and rs3749971^{T} is proven, they are not jointly passed to the off-springs in an equal ratio. On the one side, the surprising finding of an enhanced correlation between smoking and rs3749971^{T} appearance in comparison to this trait and HLA-A1-B8-DR3 is demonstrated. On the other side, a few rs3749971^{T} non-carriers were found to be HLA-A1-B8-DR3 positive. Therefore, the occurrence of the HLA-A1-B8-DR3 haplotype can be preferably determined as a second marker in addition to rs3749971^{T}. The simultaneous appearance of both markers confirms the elevated risk for initiating or continuing tobacco consumption and shifts the risk values to the maximum as stated above.

Another object of the invention is a pharmaceutical composition comprising at least one substance specifically interacting with the OR12D3 protein, which carries the single amino acid polymorphism specifying isoleucine at position 97 (SEQ ID NO: 4), optionally together with pharmaceutically tolerable adjuvants. A pharmaceutical composition in the meaning of the invention is any agent in the field of medicine, which can be used in prophylaxis, diagnosis, therapy, follow-up or aftercare of patients who have come in contact with tobacco or tobacco smoke in such a way that a pathogenic modification of their overall condition or of the condition of particular regions of the organism could establish at least temporarily. The respective dose or dosage range for administering the pharmaceutical composition according to the invention is sufficiently high in order to achieve the desired prophylactic or therapeutic effect of reducing tobacco abuse. It will be understood that the specific dose level, frequency and period of administration to any particular human will depend upon a variety of factors including the activity of the specific compound employed, the ethnicity, the age, body weight, general health, sex, diet time of administration, route of administration, rate of excretion, drug combination and the severity of the specific therapy. Using well-known means and methods, the exact dose can be determined by one of skill in the art as a matter of routine experimentation.

The specific substances are composed of biological and/or chemical structures capable to interact with the target molecule in such a manner that makes a recognition, binding and interaction possible. Preferably, the substances are mono-specific in order to guarantee an exclusive and directed interaction with the chosen single target. In particular, the pharmaceutical composition according to the invention comprises substances that are selected from the group of nucleic acids, peptides, carbohydrates, polymers, small molecules having a molecular weight between 50 and 1.000 Da and proteins. The substances are capable of discriminating between the wild type OR12D3 protein and the OR12D3^{97Ile} protein of SEQ ID NO: 4. The prior teaching of the present specification concerning the specific substances used in the method for in-vitro diagnosing a predisposition to smoking is considered as valid and applicable without restrictions to pharmaceutical compositions if these substances are expedient therein. Preferably, small and/or compact nucleic acids are provided, which do not contact other structures within a tissue or organism, respectively, or which are not attacked by the pre-mentioned ones, but specifically interact with the target molecule. The nucleic acids may be part of complexes or formulations consisting of lipids, carbohydrates, proteins or peptides, such as in the shape of a nano-capsule.

In a preferred embodiment of the invention, the pharmaceutical composition comprises an antagonist as specific substance. An antagonist is a molecule that inhibits the effect of an agonistic molecule, such as a hormone or neurotransmitter, by blocking its binding site, i.e. a receptor, without triggering any effect itself, e.g. activating a signal cascade. In the present invention, smoking, which represents a key environmental trigger for certain autoimmune diseases and other pathological conditions such as various forms of cancer, has been associated with a distinct odorant receptor allele. The translation product OR12DR3^{97Ile} (SEQ ID NO: 4) forms specific receptor/ligand complexes with such ligand molecule(s) originated from tobacco or tobacco smoke. HLA-A1-positive individuals, in contrast to those with HLA-A2 or HLA-A3, exhibit a preference for bergamot (Milinski & Wedekind, Behav. Ecol. 12, 140-149, 2001). The existence of a relationship between OR12D3^{97Ile} and this predilection for a perfume ingredient is supported by the strong linkage disequilibrium between HLA-A1 and rs3749971^{T}. The fact that many tobacco brands are scented with bergamot oil components provides an explanation for the correlation of rs3749971^{T} with smoking. That means, the modified receptor protein OR12DR3^{97Ile} allows the binding of volatile bergamot oil ingredients and triggering a signal cascade, at whose end the symptom of tobacco abuse appears. The identification of OR12DR3^{97Ile} facilitates the design of antagonists, which are used as antidotes in individuals with a predisposition to tobacco abuse. Preferably, the antagonistically acting substance is a low molecular ligand penetrating well into cells and diffusing to its destination. It is also preferred that the antagonist is a volatile substance. In a particular preferred embodiment of the present invention, the substance of the pharmaceutical composition mimics the ligand binding of a bergamot oil ingredient to the OR12DR3^{97Ile} protein, more preferably the binding of a monoterpene, most preferably the binding of linalool.

Alternatively, the pharmaceutical composition of the invention may comprise a substance specifically interacting with the rs3749971^{T} allele, or a regulator protein or a gene product thereof, or a component of a signal transduction pathway comprising the rs3749971^{T} allele or a gene product thereof, or any of the aforementioned components related to a SNP from the group represented by tag-SNP #51. Preferably, an antisense construct is used as specific substance.

Certainly, all substances of the invention may be fused or complexed with another molecule that promotes the directed transport to the destination, the incorporation and/or distribution within the target cell.

The composition of the invention is produced in a known way using common solid or liquid carriers, diluents and/or additives and usual adjuvants for pharmaceutical engineering and with an appropriate dosage depending on the intended mode of application. These pharmaceutically acceptable excipients comprise salts, buffers, fillers, chelating agents, antioxidants, solvents, bonding agents, lubricants, tablet coatings, flavor additives, flavors, preservatives and suspending agents. In the meaning of the invention, an "adjuvant" denotes every substance that enables, intensifies or modifies a specific body response to the OR12D3-specific substances if administered simultaneously, contemporarily or sequentially. Known adjuvants for injection solutions are for example aluminum compositions, such as aluminum hydroxide or aluminum phosphate, saponins, such as QS21, muramyldipeptide or muramyltripeptide, proteins, such as gamma-interferon or TNF, M59, squalen or polyols. The amount of excipient material that is combined with the active substance to produce a single dosage form varies depending upon the host treated and the particular mode of administration.

It is another object of the invention to provide a pharmaceutical composition for the prophylactic or therapeutic treatment of tobacco abuse or for the manufacture of a medicament for the prophylactic or therapeutic treatment of tobacco abuse. The composition can be either administered to prevent the initiation of tobacco consumption of a human individual and the resulting addiction in advance, or to treat the continuing abuse. Herein, monitoring is considered as kind of treatment provided that the pharmaceutical composition is sequentially administered, e.g. in order to booster the response and completely eradicate the symptoms that have arisen.

In an embodiment of the present invention, substances being mono-specific to the OR12D3^{97Ile} protein are used for the production of a medicament for the prophylactic or therapeutic treatment of tobacco abuse. The term "mono-specific" denotes a mode of binding which is characterized by the exclusive recognition of a single target. The mono-specific substances used in the present invention only recognize the OR12D3^{97Ile} protein target or variants thereof. The receptor/ligand-interaction is characterized by high affinity, high selectivity and minimal or even lacking cross-reactivity to other target molecules to exclude unhealthy and harmful impacts to the treated subject.

The substances being specific to the OR12D3 protein of SEQ ID NO: 4 are adapted in forms which are suitable for oral administration, such as tablets, film tablets, lozenges, capsules, pills, powders, solutions, dispersions, suspensions or depot forms thereof, for transdermal administration, such as solutions, suspensions, creams, ointments, perfumes, gels, emulsions or band-aids, for parental administration, such as suppositories, and for intravenous infusion, subcutaneous injection or intramuscular administration, examples for the latter three are solutions and suspensions. The substances can also be adapted for topical, transmucosal, transurethal, vaginal, rectal or pulmonary administration in the appropriate formulations given above.

Depending upon the manner of introduction, the substances may be formulated in a variety of ways as discussed below. The concentration of therapeutically active substances in the formulation may vary from about 0.1 to 100 wt %. The solution may be administered alone or in combination with other treatments. In a preferred embodiment, the substances to be injected are in a water-soluble form, such as a pharmaceutically acceptable salt, which is meant to include both acid and base addition salts. For example, these can be salts of inorganic acids such as phosphoric acid or salts of organic acids. The injection solution may also include one or more of the following: carrier proteins, such as serum albumin, buffers, stabilizing agents, coloring agents, and the like. Additives are well known in the art, and they are used in a variety of formulations.

The invention also relates to a method for treating tobacco abuse, wherein an effective amount of at least one substance being specific to the OR12D3 protein, which carries the single amino acid polymorphism specifying isoleucine at position 97 (SEQ ID NO. 4), is administered to a human in need of such treatment. The prior teaching of the invention and its embodiments is valid and applicable without restrictions to the method of treatment if expedient.

Another object of the present invention is the use of substances specifically interacting with the OR12D3^{97Ile} protein having the SEQ ID NO: 4, for the production of a medicament for the prophylactic or therapeutic treatment of tobacco abuse.

It is still another object of the invention to use substances being specific to at least one single nucleotide polymorphism (SNP) selected from the group represented by tag-SNP rs3130845, or at least one SAAP that is encoded thereby, for in-vitro diagnosis of a predisposition to smoking. Preferably, substances are used being specific to the SNP rs3749971^{T} within the OR12D3 gene or the SAAP isoleucine at position 97 within the OR12D3 protein for in-vitro diagnosis of a predisposition to smoking. The substances are selected from the group of nucleic acids, peptides, carbohydrates, polymers, small molecules having a molecular weight between 50 and 1.000 Da, and proteins, but preferably nucleic acids. More preferably, the specific substances are at least one DNA sequence of the SEQ ID NOs: 1 to 3 to be used for in-vitro diagnosis. The prior teaching of the present specification concerning the diagnostic method or the pharmaceutical composition, respectively, is considered as valid and applicable without restrictions to the use of such substances in in-vitro diagnosis, or prophylaxis and treatment of tobacco abuse if expedient.

Further, the invention may be practiced as a kit comprising substances being specific to at least one single nucleotide polymorphism (SNP) selected from the group represented by tag-SNP rs3130845, or at least one SAAP that is encoded thereby, particularly in order to perform the inventive method of diagnosing a predisposition to smoking. The kit favorably comprises substances being specific to the SNP rs3749971^{T} within the OR12D3 gene or the SAAP specifying isoleucine at position 97 within the OR12D3 protein, Thus, the invention includes an article that comprises written instructions or directs the user to written instructions for how to practice the method of the invention. Preferably, the specific substances of the kit are at least one DNA sequence of the SEQ ID NOs: 1 to 3. In an embodiment, the kit further comprises a reporter moiety or a reporter apparatus, preferably a fluorophore or a field-effect transistor. Additionally, the kit may comprise a nucleic acid extracting reagent for isolating a nucleic acid containing the OR12D3 gene. The prior teaching of the present specification concerning the diagnostic method is considered as valid and applicable without restrictions to the kit if expedient.

The invention also relates to the DNA sequences having the SEQ ID NOs: 1 to 3.

Object of the invention is also a method for screening substances, which reduce the predilection for ingredients of tobacco, tobacco mixtures and/or tobacco smoke, comprising the steps of providing at least two subjects of a non-human organism being capable of expressing the OR12D3 protein, which carries an SAAP specifying isoleucine at position 97 (SEQ ID NO. 4), administering substances, which are to be screened, to a subset of the subjects, comparing the predilection for ingredients of tobacco smoke in subjects to which substances have been administered, with the predilection for ingredients of tobacco smoke in subjects to which no substances have been administered, and detecting the specific binding of substances to the OR12D3^{97Ile} protein.

The inventive method makes the identification and analysis of substances possible, which are responsible for the reduction of tobacco affection. The non-human organism is preferably a mammal, more preferably species such as mice or rats that may be genetically modified. However, the non-human organism may also represent a cell sample, which refers to primary cells or genetically engineered cells, provided that a suitable assay allows the determination of the predilection for ingredients of tobacco, tobacco mixture and/or tobacco smoke in cells. Such cells are capable of expressing OR12D3^{97Ile}, e.g. by transfection with appropriate vectors harboring the matching gene with rs3749971^{T} or parts thereof. Preferably, the recombinant cells are of eukaryotic origin. The total number or amount, respectively, is divided into multiple portions. At least two portions are provided; one is used for screening while the other one serves as negative control. Preferably, the number of portions for screening exceeds the number of control portions. Numerous cell portions can be subjected to a high-throughput screening. Technology suitable to carry out said test is known to one skilled in the art and described, for instance, in the article by Katada et al. (J. Neurosci. 25, 1806-1815, 2006), which is incorporated as reference in the disclosure of the invention hereby.

The substances to be screened in the inventive method are not restricted in any way. In an embodiment of the invention, the substances are selected from the group of nucleic acids, peptides, carbohydrates, polymers, small molecules having a molecular weight between 50 and 1.000 Da, and proteins. In a preferred embodiment of the invention, volatile substances are administered. The prior teaching of the present specification concerning the pharmaceutical composition and their active substances for the prophylactic or therapeutic treatment of human tobacco abuse is considered as valid and applicable without restrictions to the method for screening such substances which reduce the predilection for ingredients of tobacco smoke if expedient.

The substances are often available in libraries. It is possible to contact rats or mice, for example, with the substance candidates by injection, infusion, oral or rectal intake. It is preferred to incubate a single substance within a distinct portion of the non-human organisms. However, it is also possible to investigate the cooperative effect of substances by administering at least two substances within one subset. A further subset of subjects that are susceptible to tobacco or tobacco smoke is simultaneously observed in the absence of the substances. The incubation process of subjects depends on various parameters, e.g. the organism type and the sensitivity of detection, whose optimization follows routine procedures known to those skilled in the art.

The identification of effective substances in the meaning of the invention is directly performed by determining the degree of reduction of predilection to tobacco smoke ingredients. Suitable tests are known to those skilled in the art or can be easily designed as a matter of routine. For example, the subset of the non-human organism being subjected to substances is contacted to those components of bergamot oil that are used to scent tobacco brands (Baker et al., Food Chem. Toxicol. 42, Suppl.: S3-S37, 2004) and subsequently, distinct signals in the signal cascade via the protein OR12D3 are measured. The determination is performed at a specified moment and correlated to the signal strength at the beginning of the experiment and the negative control. The reduction or even absence of signals reflects a potentially active antagonist.

Among those substances being revealed to reduce the tobacco predilection each or some representatives are selected for further analysis. Preferably, the substances showing the greatest discrepancy to the control are chosen. They are analyzed for specificity to OR12DR3^{97Ile} to exclude another signal transduction that is not initiated by OR12DR3^{97Ile} receptor binding, and additionally tested for cross-reactivity in order to prevent adverse reactions or other effects by linked pathways if simultaneous docking to further receptors occurs. Several methods are known in the field of the art for detecting specific and/or mono-specific binding, such as gel shift experiments, Biacore measurements, X-ray structure analysis, competitive binding studies, and the like. In a preferred embodiment of the present invention, the mono-specific binding to OR12DR3^{97Ile} of substances reducing tobacco predilection is detected.

In the scope of the present invention, a method for in-vitro diagnosing a predisposition to smoking by using specific substances to the SNP rs3749971^{T} within the OR12D3 gene or the SAAP of OR12D3^{97Ile} , or even further SNPs from the group represented by tag-SNP rs3130845 or the SAAP encoded thereby, and detecting the specific incubation products is provided for the first time. The individual genetic constitution plays a role in initiating and continuing tobacco consumption. The present invention teaches an HLA-linked OR allele that is associated with tobacco abuse and implies that the rs3749971^{T} allele is involved in the smoking-induced aggravation of certain autoimmune diseases that can be observed in patients carrying A1-B8-DR3. Given the fact that A1-B8-DR3 occurs with a frequency of 5-10 % in Caucasians, the rs3749971^{T} gene polymorphism is very suitable for large-scale screening tests, particularly among young people. In doing so, individuals are identified with an elevated risk for initiating or continuing tobacco consumption and for acquiring disorders, such as lung cancer or coronary artery disease, that are clearly connected with smoking. Actions serving the primary prevention or put somebody off smoking are of great significance for health care policy and insurance medicine. In this context, the test is of special benefit for health and life insurance companies in respect of risk appraisal and may exert an influence on cash flow underwriting. The diagnostic method of the invention can be performed in a simple and fast manner. In addition, the appropriate kit is cost-efficiently produced. The present method favorably goes without a blood collection since a few cells of oral mucosa are sufficient for testing.

The pharmaceutical composition of the invention allows a therapeutic suppression of tobacco predilection causing a direct and immediate reduction of abuse symptoms. The pharmaceutical composition advantageously attacks the docking site of the OR12D3 receptor that is abnormally modified by a single amino acid exchange from threonine to isoleucine at position 97. The SAAP impacts the ligand specificity to be utilized by the substances underlying the pharmaceutical invention. The substances are characterized by a high affinity, specificity and stability, low manufacturing costs and convenient handling due to the small size. These features form the basis for a reproducible detection, wherein the lack of cross-reactivity is included, and for a reliable and safe blocking of the OR12D3^{97Ile} binding site.

It is to be understood that this invention is not limited to the particular methods, pharmaceutical compositions, uses or kits described herein, as such matter may, of course, vary. It is also to be understood that the terminology used herein is for the purpose of describing particular embodiments only and is not intended to limit the scope of the present invention, which is only defined by the appended claims. As used herein, including the appended claims, singular forms of words such as "a," "an," and "the" include their corresponding plural referents unless the context clearly dictates otherwise. Thus, e.g., reference to "a substance" includes one or more different substances and reference to "a method" includes reference to equivalent steps and methods known to a person of ordinary skill in the art, and so forth. Unless otherwise defined, all technical and scientific terms used herein have the same meaning as commonly understood by a person of ordinary skill in the art to which this invention belongs.

Although methods and materials similar or equivalent to those described herein can be used in the practice or testing of the present invention, suitable examples are described below. The following examples are provided by way of illustration and not by way of limitation. Within the examples, standard reagents and buffers that are free from contaminating activities (whenever practical) are used. Nucleotide diversity (π) was calculated as described by Nei, M. (Molecular Evolutionary Genetics. 1987, Columbia Univ. Press, New York) using the software DNAsp (http://www.ub.es/dnasp). Two-sided Fisher's exact test was employed for all association analyses, with a 1% level of significance. The Bonferroni correction for multiple comparisons was applied to adjust p-values only of the in-silico analyses.

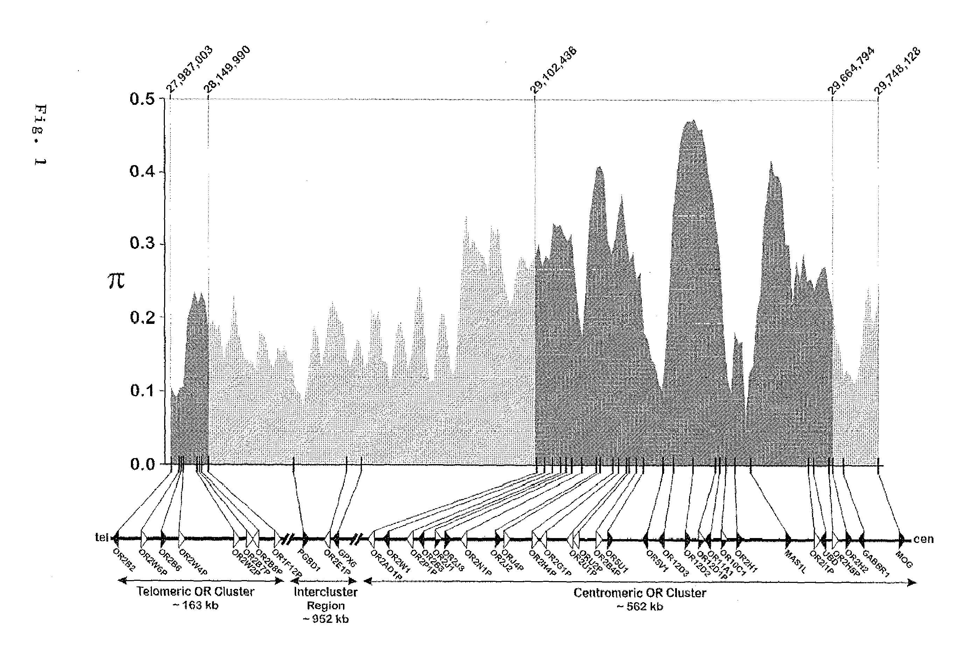

Fig. 1 shows the diversity of 1170 SNPs in a region of the xHLA complex. This region encompasses the two odorant receptor (OR) clusters on chromosome 6p (between the genes OR2B2 and MOG). The sliding windows plot (18 bases wide, stepping 6 bases after each measurement) shows the average number of nucleotide differences per site (π). OR clusters are plotted in dark gray, and light gray is used for the regions outside the OR clusters. The ticks under the graph mark the position of OR genes and pseudogenes in relation to the plot. Above the graph, the NCBI 36 coordinates (bp) of selected loci are depicted. The line underneath the graph shows the OR genes/pseudogenes at their approximate chromosomal locations. Triangles indicate transcriptional orientation, and are filled in black (genes), white (pseudogenes) or in both colors (haplotype-dependent genes/pseudogenes). tel: telomere; cen: centromere.

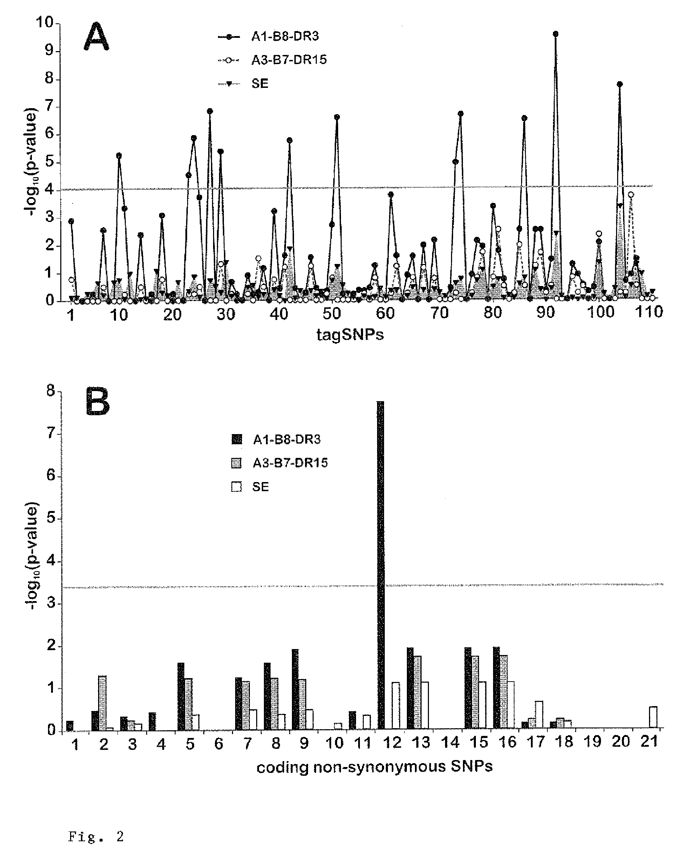

Fig. 2 shows the allelic association of two sets of SNPs from the OR clusters within the xHLA complex. 110 tagSNPs in panel A and 21 coding, non-synonymous SNPs in panel B, from the genomic region between the genes OR2B2 and MOG within the A1-B8-DR3, A3-B7-DR15, and SE haplotypes of the xHLA were analyzed. Panel A: the gray horizontal line marks the Bonferroni threshold for 110 tests with a significance level of 1%. Panel B: the strong association of SNP 12 (rs3749971) with A1-B8-DR3 is a consequence of the presence of the OR12D3^{T} allele in twenty haplotypes in the CEPH panel, of which nine belong to the group of the eleven A1-B8-DR3 haplotypes. The gray horizontal line marks the Bonferroni threshold for 21 tests with a significance level of 1%.

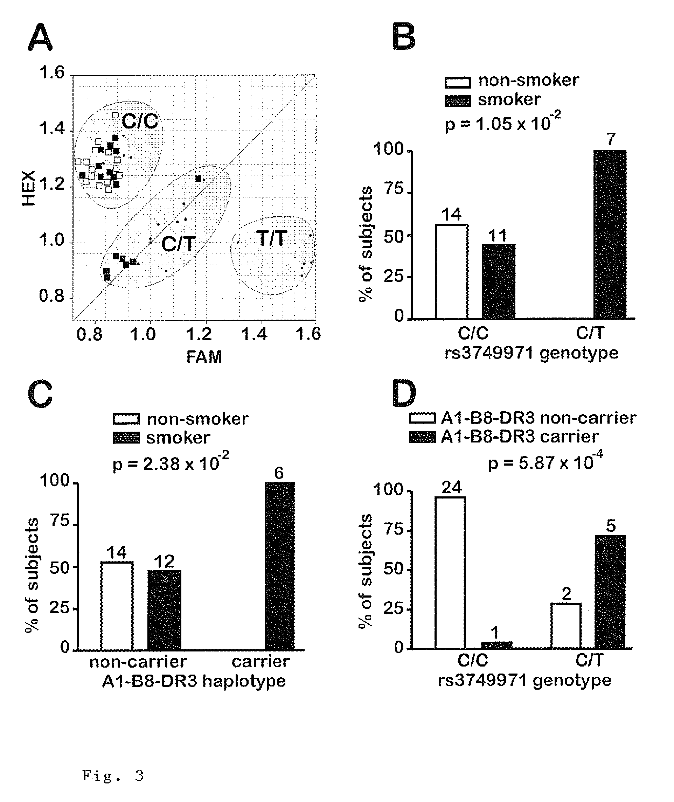

Fig. 3 shows real-time genotyping of rs3749971. HEX and FAM (panel A) are primer fluorophores marking the T- and the C-allele-primers, respectively. Values indicate the relative number of PCR-cycles necessary to reach the genotyping threshold for each allele, normalized according to typed reference samples. HEX-values around 1 (or below) indicate the presence of the T-allele, and the same is true for FAM-values indicating the presence of the C-allele. Filled squares: 17 smokers; open squares: 15 non-smokers; dots: controls. Panels B to D: association of smoking behavior with rs3749971 genotypes (panel B), with the carrier state of A1-B8-DR3 (panel C), as well as A1-B8-DR3 and rs3749971 genotypes (panel D) in 32 unrelated Hungarian females. The number of individuals in each category is indicated above the corresponding bars, as well as the P values as determined by Fisher's exact test.

### EXAMPLE 1: In-silico analyses

All in-silico analyses were based on data from 180 founder (unrelated, grandparental) chromosomes from the Centre d'Etude du Polymorphisme Humain (CEPH) collection. The phasing of haplotypes was based on family information. Further details on marker selection, CEPH subjects and on their DNA typing are given by Miretti et al. (Am. J. Hum. Genet. 76, 634-646, 2005) as well as de Bakker et al. (Nat. Genet. 38, 1166-1172, 2006).

All 1170 SNPs (Fig. 1, 2 and Tables 1, 2) were typed through the Illumina's Golden Gate genotyping platform in the Wellcome Trust Sanger Institute, Hinxton, Cambridge, UK. DNA samples were the 180 founder CEPH chromosomes. From this set of markers, two subsets of SNPs were chosen, (a) 110 tag-SNPs capturing the haplotypic information from 1170 SNPs spanning the OR region of the xHLA (Fig. 2A, Tables 1, 2) and (b) 21 coding, non-synonymous SNPs HapMap-validated (Fig. 2B and Table 3). Pair-wise tagging of SNPs was performed through HAPLOVIEW v 3.32, using an LD coefficient (*r*²) threshold of 0.8. Loci with a minor allele frequency of less than 1%, those not conforming to Hardy-Weinberg equilibrium, and those not reported by NCBI-dbSNP and ENSEMBL databases, were excluded. The chromosomal regions captured by different tag-SNPs may overlap.

To uncover HLA haplotype-dependent polymorphisms in the chromosomal segment encompassing the telomeric and the centromeric OR gene clusters, three approaches were used to analyze the region between the genes OR2B2 and MOG: determination of nucleotide diversity and analyses of the haplotype-dependent association either of tagSNPs representative for all SNPs within the region, or of non-synonymous, coding SNPs between the loci OR2B2 and MOG.

The degree of nucleotide diversity as assessed by 1170 SNPs (Fig. 1 and Table 1) revealed that it is highest between the genes OR12D2 and OR10C1, the most polymorphic loci within this cluster. The SNP diversity outside of the OR clusters did not differ substantially from that within the clusters. SNP densities within the telomeric (3.88/10 kb) and the centromeric OR clusters (8.77/10 kb) were relatively high when compared with other genomic regions.

110 tagSNPs were selected, which are representative of all 1170 SNPs (Supplementary Table 1) and independently tested for association with three groups of haplotypes: A1-B8-DR3 (over-represented in individuals with Graves' disease), A3-B7-DR15 (over-represented in multiple sclerosis patients) as well as HLA-DR "shared epitope" (SE) haplotypes. The latter comprise HLA haplotypes that carry the HLA-DRB1*01, DRB1*04 or DRB1*10 alleles, and are over-represented in individuals who smoke, possess antibodies against citrullinated auto-antigens and suffer from rheumatoid arthritis. Within the panel of 180 xHLA haplotypes, eleven A1-B8-DR3, five A3-B7-DR15, and 59 HLA-DR SE haplotypes were found. Fig. 2A shows that no significant association between any of the tagSNPs and the 59 HLA-DR SE haplotypes was observed if compared with the remaining 121 non-SE haplotypes. A similar result was obtained with regard to the A3-B7-DR15 haplotypes. For twelve tag-SNPs (representing 81 tagged SNPs, Tables 1 and 2) of the A1-B8-DR3 haplotypes, however, a significant correlation was found after Bonferroni correction when the eleven A1-B8-DR3 haplotypes were compared with the 169 non-A1-B8-DR3 haplotypes (Fig. 2A). Only one of these 81 tagged SNPs is a coding, non-synonymous SNP (rs3749971), within the gene OR12D3. In A1-B8-DR3 haplotypes, the respective allele (rs3749971^{T}) is responsible for a Thr→Ile exchange at amino acid position 97 within the OR12D3 protein. The other 80 SNPs lead either to synonymous exchanges or are located in intergenic or in intronic regions.

In order to identify which coding, non-synonymous SNPs between the genes OR2B2 and MOG were characteristic for the A1-B8-DR3, A3-B7-DR15 and the SE haplotypes, the association of alleles of all 21 coding, non-synonymous SNPs (Table 3) from the region depicted in Fig. 1 was determined. Statistical significance was obtained only for rs3749971^{T}, when assessing its association with A1-B8-DR3 (Fig. 2B). This is a consequence of the occurrence of the rs3749971^{T} allele in 20/180 haplotypes. Of these 20, nine haplotypes belonged to the A1-B8-DR3 group (N = 11), another nine carried centromerically truncated versions of A1-B8-DR3 (e.g., with retention of HLA-A*0101 and -B*0801, but loss of HLA-DRB1*03), while the last two did not share any HLA class I and II alleles with A1-B8-DR3. Two atypical A1-B8-DR3 haplotypes were found that exhibited the rs3749971^{C} allele due to distinct recombinations in the centromeric HLA-linked OR cluster that had led to elimination of the rs3749971^{T} allele. Therefore, both types of analyses of the xHLA haplotypes in the CEPH panel (Fig. 2) resulted in the identification of the same coding, non-synonymous SNP allele (rs3749971^{T}) that is significantly associated with A1-B8-DR3 (p = 1.84 x 10⁻⁸).

### EXAMPLE 2: Genotyping of rs3749971 by real-time PCR

The genotyping of the rs3749971 SNP was performed in a sub-sample of the Hungarian cohort in which a correlation had been found between smoking and A1-B8-DR3 haplotypes by Füst et al. (Int. Immunol. 16, 1507-1514, 2004). This sub-sample consisted of 32 Hungarian female Caucasians with an average age of 46.75 years ranging from 24 to 76 years old (Fig. 3A). These individuals differ in their smoking behavior and do not suffer from an autoimmune disease. Women who carried the C4A*Q0 (mono-S) genotype as well as the RAGE 429C, HSP-70 1267G and TNF -308A alleles were considered carriers of the A1-B8-DR3 haplotype. Other genotyping of these subjects, details on registration of smoking habits, preparation of genomic DNA, as well as on informed consent from the cohort are given by Füst et al. (Int. Immunol. 16, 1507-1514, 2004).

The PCR reactions were performed using a Stratagene real-time PCR instrument with the following cycle conditions: 1 cycle of 94°C for 1 min and 80 cycles of 94°C for 20 s, 62°C for 20 s, 72°C for 30 s, and 78°C for 10 s. Fluorescence was measured during the 62°C step. 20-µL PCR reactions contained 100 ng human genomic DNA; 1.5pmol C- or T-specific reverse primers (details see below); 3pmol forward primer (rs3749971-For;AGCGAAGAGGATTGCAGATGGC); 2 µl Genetherm polymerase buffer; 0.7mM each of dATP, dCTP, dGTP and dTTP; 2 mM MgCl₂; and 2U of GenTherm^{™} Taq Polymerase. Allele specific primers were designed as molecular beacons (Jordens et al., J. Virol. Meth. 89, 29-37, 2000) (rs3749971-C Fam-atacagcCTATATCTTTTCTAGGCTGTAT_{BHQ}CAC and rs3749971-T Hex-atacagcCTATATCTTTTCTAGGCTGTAT_{BHQ}CAT) labeled either with FAM™ (6-Carboxyfluorescein) or HEX™ (4,7,2',4',5',7'-hexachloro-6-carboxyfluorescein-) and quenched with Black Hole Quencher (BHQ^{™}) attached to a T natively present within the primer binding site. Seven bases were added to the 5' end (small letters) to allow the formation of a 29 bp hairpin with a complementary region of 10 bp to ensure fluorescence quenching of the unused primer. To assess the reproducibility of the genotyping approach, control DNA of 7 individuals (including 3 CEPH samples) was typed 15 times independently with 100% reproducibility.

The correlation between smoking and rs3749971^{T} was slightly stronger (p = 1.05 x 10⁻²) (Fig. 3B) than the correlation between this trait and A1-B8-DR3 (p = 2.38 x 10⁻²) (Fig. 3C). Seven and 25 subjects, respectively, were rs3749971^{C/T} and rs3749971^{C/C} carriers. A strong association (p = 5.87 x 10⁻⁴) between the rs3749971^{T} allele and the A1-B8-DR3 haplotype was observed, as five out of seven of the rs3749971^{T} carriers but only 1/25 of the non-carriers were found to be A1-B8-DR3 positive (Fig. 3D).

### EXAMPLE 3: Smelling test designed to identify individuals expressing the OR12D3^{97Ile} variant

Each individual is provided with three coded glass vials each of which contains a 3.5 cm long smelling strip (as commonly used in perfumology) that is fixed to the plug. The strip of the first vial is supplied with two drops of a solution containing a ligand for the OR12D3^{97Ile} protein, e.g. a monoterpene such as linalool. The strips within the second and third vials will contain control substances that are known not to interact with the OR12D3^{97Ile} protein, e.g. isovaleric acid or acetic acid. The strip can be removed with the plug from the vial untouched by the smeller.

The test must be directed at obtaining the scorings dependent on the presence or absence of the OR12D3^{97Ile} protein within the nasal mucosa of a given smeller. After smelling the strip from a given vial, the individuals participating in the test will be asked: "Can you smell anything at all on the strip? As how pleasant do you perceive this scent as a potential ingredient of a perfume/aftershave that you would like to use for yourself? (mark between unpleasant and pleasant)." Potential variation in the results can be minimized by carrying out the study in a standardized fashion, e.g. with regard to smelling time and intensity.

Individuals that cannot express the OR12D3^{97Ile} protein because they do not carry the rs3749971^{T} allele (i.e. those who are homozygous for the rs3749971^{C} allele) are expected to lack recognition of the test substance in the first vial, while they should be able to recognize the substances on the strips from the other vials. Individuals expressing the OR12D3^{97Ile} protein, i.e. those that are either heterozygous or homozygous for the rs3749971^{T} allele, are expected to be able to smell also the substance on the strip from the first vial, while their response will be identical to rs3749971^{C} - homozygous individuals with regard to the strips from the other vials.

This smelling test is of a purely diagnostic nature. It will only be able to indicate the presence of the rs3749971^{T} allele.

**Table 1: List of 1170 SNPs analysed in the study, with their SNP IDs according to NCBI's dbSNP database , and coordinates (as from build 36). Loci used as tagSNPs are indicated, and the SNPs captured by each tagSNPs are given in Table S2**

| **Order** | **dbsNP ID** | **Coordinate (NCBI 36)** | **tagSNP** |
|---|---|---|---|
| 0001 | rs2394015 | 27978337 | |
| 0002 | rs200945 | 27989883 | |
| 0003 | rs201910 | 27991536 | |
| 0004 | rs13218875 | 27991991 | |
| 0005 | rs9468254 | 27992895 | |
| 0006 | rs1015075 | 27995254 | |
| 0007 | rs6927241 | 27997313 | |
| 0008 | rs6933825 | 27998610 | |
| 0009 | rs156737 | 28003192 | |
| 0010 | rs9468256 | 28003483 | |
| 0011 | rs12663899 | 28007558 | tagSNP #1 |
| 0012 | rs7760871 | 28009020 | tagSNP #2 |
| 0013 | rs7742529 | 28010848 | |
| 0014 | rs7742858 | 28010971 | |
| 0015 | rs10456357 | 28011446 | |
| 0016 | rs6910968 | 28012160 | |
| 0017 | rs2893937 | 28018029 | |
| 0018 | rs276366 | 28035460 | |
| 0019 | rs9295753 | 28036424 | tagSNP #3 |
| 0020 | rs276364 | 28039599 | |
| 0021 | rs276363 | 28041280 | |
| 0022 | rs7769416 | 28056553 | |
| 0023 | rs9380031 | 28062677 | tagSNP #4 |
| 0024 | rs7748445 | 28069926 | tagSNP #5 |
| 0025 | rs1143887 | 28072802 | |
| 0026 | rs149901 | 28073482 | |
| 0027 | rs156743 | 28075068 | |
| 0028 | rs12333142 | 28076983 | |
| 0029 | rs149946 | 28078010 | |
| 0030 | rs6939591 | 28087157 | |
| 0031 | rs10484402 | 28087604 | tagSNP #6 |
| 0032 | rs9368540 | 28089652 | |
| 0033 | rs149971 | 28090131 | |
| 0034 | rs149972 | 28091206 | |
| 0035 | rs149973 | 28091592 | |
| 0036 | rs149974 | 28093075 | |
| 0037 | rs6456804 | 28093367 | |
| 0038 | rs2840222 | 28093541 | |
| 0039 | rs2893947 | 28094129 | |
| 0040 | rs149975 | 28094319 | |
| 0041 | rs9368545 | 28107023 | |
| 0042 | rs1529749 | 28108340 | |
| 0043 | rs3926997 | 28108508 | |
| 0044 | rs149941 | 28109012 | |
| 0045 | rs149942 | 28109589 | |
| 0046 | rs149943 | 28110367 | |
| 0047 | rs185741 | 28111250 | |
| 0048 | rs149897 | 28114629 | tagSNP #7 |
| 0049 | rs149896 | 28116503 | |
| 0050 | rs175954 | 28119564 | |
| 0051 | rs149900 | 28122576 | |
| 0052 | rs149962 | 28123897 | |
| 0053 | rs149965 | 28126668 | |
| 0054 | rs9393879 | 28126923 | |
| 0055 | rs203888 | 28129568 | |
| 0056 | rs9468274 | 28134056 | |
| 0057 | rs6903723 | 28135789 | |
| 0058 | rs13198131 | 28137003 | |
| 0059 | rs9380046 | 28138478 | |
| 0060 | rs7741570 | 28141484 | |
| 0061 | rs149952 | 28141864 | |
| 0062 | rs149957 | 28144999 | |
| 0063 | rs9380047 | 28145872 | |
| 0064 | rs203881 | 28146096 | |
| 0065 | rs4713135 | 28147565 | |
| 0066 | rs5021186 | 28149070 | |
| 0067 | rs6913038 | 28152735 | tagSNP #8 |
| 0068 | rs3956922 | 28153995 | |
| 0069 | rs172164 | 28154457 | |
| 0070 | rs203876 | 28154652 | |
| 0071 | rs12211199 | 28155049 | |
| 0072 | rs203877 | 28156603 | |
| 0073 | rs9393885 | 28157988 | |
| 0074 | rs427348 | 28166344 | tagSNP #9 |
| 0075 | rs1853097 | 28166614 | |
| 0076 | rs175955 | 28167713 | |
| 0077 | rs13197574 | 28168218 | |
| 0078 | rs3823180 | 28169723 | |
| 0079 | rs203893 | 28170045 | |
| 0080 | rs9380051 | 28172070 | |
| 0081 | rs169433 | 28172715 | |
| 0082 | rs203891 | 28175777 | |
| 0083 | rs10946952 | 28181443 | |
| 0084 | rs203885 | 28184865 | |
| 0085 | rs203884 | 28185353 | |
| 0086 | rs6924324 | 28190345 | |
| 0087 | rs9368554 | 28190690 | |
| 0088 | rs1654775 | 28191404 | |
| 0089 | rs4713137 | 28191500 | |
| 0090 | rs6456807 | 28194055 | |
| 0091 | rs1770132 | 28194644 | |
| 0092 | rs478616 | 28196359 | |
| 0093 | rs7747772 | 28198641 | |
| 0094 | rs539474 | 28200285 | |
| 0095 | rs6922063 | 28202345 | |
| 0096 | rs1770129 | 28203125 | |
| 0097 | rs4713140 | 28205172 | tagSNP #10 |
| 0098 | rs1383394 | 28205474 | |
| 0099 | rs6914592 | 28208091 | |
| 0100 | rs4713141 | 28209657 | |
| 0101 | rs6941992 | 28214120 | |
| 0102 | rs4713142 | 28214326 | tagSNP #11 |
| 0103 | rs4713145 | 28214806 | |
| 0104 | rs3757188 | 28215336 | |
| 0105 | rs3757186 | 28215641 | |
| 0106 | rs1904841 | 28216064 | |
| 0107 | rs1904840 | 28216211 | |
| 0108 | rs2791331 | 28216691 | |
| 0109 | rs2277103 | 28217612 | |
| 0110 | rs868987 | 28218127 | |
| 0111 | rs1890809 | 28218457 | |
| 0112 | rs2791333 | 28219093 | |
| 0113 | rs1225715 | 28221352 | |
| 0114 | rs4713149 | 28227777 | |
| 0115 | rs2622318 | 28227865 | |
| 0116 | rs6908414 | 28229807 | |
| 0117 | rs6932313 | 28230328 | |
| 0118 | rs1225604 | 28230544 | |
| 0119 | rs1150666 | 28231907 | |
| 0120 | rs1150667 | 28232042 | |
| 0121 | rs6928131 | 28233428 | |
| 0122 | rs1225603 | 28233606 | |
| 0123 | rs9393897 | 28235689 | |
| 0124 | rs1225618 | 28237692 | |
| 0125 | rs1150671 | 28239022 | |
| 0126 | rs1564442 | 28240405 | tagSNP #12 |
| 0127 | rs1225713 | 28241091 | |
| 0128 | rs9283884 | 28243639 | |
| 0129 | rs1144707 | 28243978 | |
| 0130 | rs3173443 | 28245006 | |
| 0131 | rs4713152 | 28245433 | |
| 0132 | rs9393902 | 28245709 | tagSNP #13 |
| 0133 | rs1150675 | 28245996 | |
| 0134 | rs1150677 | 28248113 | |
| 0135 | rs1150678 | 28250255 | |
| 0136 | rs2840214 | 28250520 | |
| 0137 | rs6904045 | 28252208 | |
| 0138 | rs6904277 | 28252423 | |
| 0139 | rs1233667 | 28254653 | |
| 0140 | rs9295761 | 28255966 | |
| 0141 | rs1225591 | 28256731 | |
| 0142 | rs1225593 | 28258498 | |
| 0143 | rs1150683 | 28263293 | |
| 0144 | rs12205680 | 28265083 | |
| 0145 | rs1237873 | 28266933 | |
| 0146 | rs1233704 | 28274902 | |
| 0147 | rs1233702 | 28275869 | |
| 0148 | rs1233701 | 28276705 | |
| 0149 | rs768484 | 28277094 | |
| 0150 | rs1233698 | 28277602 | |
| 0151 | rs735765 | 28278276 | |
| 0152 | rs1233708 | 28281198 | tagSNP #14 |
| 0153 | rs1150696 | 28283528 | |
| 0154 | rs1150697 | 28283615 | |
| 0155 | rs1233666 | 28284394 | |
| 0156 | rs1150700 | 28290349 | |
| 0157 | rs1150701 | 28291865 | |
| 0158 | rs1233664 | 28293705 | tagSNP #15 |
| 0159 | rs1736894 | 28294533 | |
| 0160 | rs1736892 | 28294957 | |
| 0161 | rs1736891 | 28295080 | |
| 0162 | rs11967622 | 28296593 | |
| 0163 | rs1736890 | 28297368 | |
| 0164 | rs12211332 | 28298142 | |
| 0165 | rs3757183 | 28299838 | |
| 0166 | rs9461443 | 28302608 | |
| 0167 | rs11751492 | 28303299 | |
| 0168 | rs1150706 | 28304593 | |
| 0169 | rs2299031 | 28304697 | |
| 0170 | rs1150707 | 28305584 | |
| 0171 | rs1679723 | 28307579 | |
| 0172 | rs1233663 | 28308211 | |
| 0173 | rs7206 | 28309117 | |
| 0174 | rs6918043 | 28309447 | |
| 0175 | rs11752073 | 28312772 | tagSNP #16 |
| 0176 | rs7769054 | 28317176 | |
| 0177 | rs1736889 | 28317269 | |
| 0178 | rs967005 | 28318667 | |
| 0179 | rs7757215 | 28320468 | |
| 0180 | rs1150712 | 28321218 | |
| 0181 | rs13200462 | 28326178 | |
| 0182 | rs1679732 | 28329243 | |
| 0183 | rs10456362 | 28329795 | |
| 0184 | rs2185955 | 28330959 | |
| 0185 | rs2394048 | 28331170 | |
| 0186 | rs7750106 | 28331659 | tagSNP #17 |
| 0187 | rs12000 | 28335415 | |
| 0188 | rs1635 | 28335583 | |
| 0189 | rs1679705 | 28337175 | |
| 0190 | rs1778508 | 28337860 | |
| 0191 | rs1778507 | 28338696 | |
| 0192 | rs9468322 | 28339222 | |
| 0193 | rs2799076 | 28340257 | |
| 0194 | rs4713154 | 28340648 | |
| 0195 | rs4713155 | 28342381 | |
| 0196 | rs2799077 | 28342576 | |
| 0197 | rs1778484 | 28348777 | |
| 0198 | rs1419183 | 28350773 | |
| 0199 | rs1150725 | 28351679 | |
| 0200 | rs1150724 | 28358215 | tagSNP #18 |
| 0201 | rs2142731 | 28358892 | |
| 0202 | rs11756111 | 28360838 | tagSNP #19 |
| 0203 | rs1150722 | 28361511 | |
| 0204 | rs12526248 | 28362667 | |
| 0205 | rs13211507 | 28365356 | |
| 0206 | rs2039813 | 28373455 | |
| 0207 | rs2142730 | 28374128 | |
| 0208 | rs2223287 | 28375287 | |
| 0209 | rs2281043 | 28376476 | |
| 0210 | rs6456811 | 28378026 | tagSNP #20 |
| 0211 | rs1062169 | 28378230 | |
| 0212 | rs1339899 | 28379581 | |
| 0213 | rs4711166 | 28388516 | |
| 0214 | rs6901990 | 28389327 | |
| 0215 | rs7759855 | 28390842 | |
| 0216 | rs13195487 | 28395454 | |
| 0217 | rs742107 | 28398693 | |
| 0218 | rs707907 | 28399219 | |
| 0219 | rs4711167 | 28402867 | tagSNP #21 |
| 0220 | rs6902583 | 28403512 | |
| 0221 | rs9468343 | 28407130 | |
| 0222 | rs6456812 | 28407861 | |
| 0223 | rs6910120 | 28408295 | |
| 0224 | rs7752721 | 28409146 | |
| 0225 | rs4713161 | 28410483 | |
| 0226 | rs1416920 | 28410763 | |
| 0227 | rs1416919 | 28410863 | |
| 0228 | rs723476 | 28413083 | |
| 0229 | rs6908459 | 28413472 | |
| 0230 | rs213244 | 28414887 | tagSNP #22 |
| 0231 | rs2108926 | 28416726 | tagSNP #23 |
| 0232 | rs1119211 | 28417115 | tagSNP #24 |
| 0233 | rs6918631 | 28420435 | |
| 0234 | rs6929449 | 28421781 | |
| 0235 | rs213240 | 28423854 | |
| 0236 | rs9468346 | 28425693 | |
| 0237 | rs6921919 | 28433180 | |
| 0238 | rs213233 | 28433654 | |
| 0239 | rs1555047 | 28436662 | |
| 0240 | rs1555046 | 28437016 | |
| 0241 | rs213230 | 28438243 | |
| 0242 | rs213229 | 28438666 | |
| 0243 | rs213228 | 28439231 | tagSNP #25 |
| 0244 | rs7773018 | 28439310 | |
| 0245 | rs11751928 | 28443357 | |
| 0246 | rs7773051 | 28448295 | |
| 0247 | rs7449675 | 28448851 | |
| 0248 | rs6916243 | 28450900 | |
| 0249 | rs6938371 | 28451712 | |
| 0250 | rs7769304 | 28453760 | |
| 0251 | rs7774981 | 28454889 | |
| 0252 | rs2072319 | 28456639 | |
| 0253 | rs7765637 | 28456876 | |
| 0254 | rs1556957 | 28457905 | |
| 0255 | rs3799500 | 28460636 | tagSNP #26 |
| 0256 | rs7763846 | 28461760 | |
| 0257 | rs2859379 | 28466174 | |
| 0258 | rs2232428 | 28467679 | |
| 0259 | rs2232426 | 28468638 | tagSNP #27 |
| 0260 | rs2232425 | 28468817 | |
| 0261 | rs2041230 | 28473494 | |
| 0262 | rs1005125 | 28475334 | |
| 0263 | rs6907950 | 28478225 | |
| 0264 | rs6908137 | 28478372 | |
| 0265 | rs4254981 | 28479381 | |
| 0266 | rs2531828 | 28481769 | |
| 0267 | rs2859361 | 28483586 | |
| 0268 | rs4382246 | 28488227 | |
| 0269 | rs6899389 | 28489119 | |
| 0270 | rs3757181 | 28489875 | |
| 0271 | rs2394098 | 28490731 | |
| 0272 | rs6922169 | 28490911 | |
| 0273 | rs10484540 | 28495422 | |
| 0274 | rs7451680 | 28497230 | |
| 0275 | rs1024629 | 28498379 | |
| 0276 | rs1015690 | 28498505 | tagSNP #28 |
| 0277 | rs3800328 | 28498822 | |
| 0278 | rs2859365 | 28499444 | |
| 0279 | rs7740351 | 28507391 | |
| 0280 | rs7765989 | 28508274 | |
| 0281 | rs2859366 | 28508416 | |
| 0282 | rs6930745 | 28510082 | |
| 0283 | rs11752919 | 28511582 | |
| 0284 | rs2531801 | 28512676 | |
| 0285 | rs11750984 | 28519426 | |
| 0286 | rs2859367 | 28522145 | |
| 0287 | rs6939966 | 28523864 | tagSNP #29 |
| 0288 | rs2531808 | 28528763 | |
| 0289 | rs2531810 | 28529744 | |
| 0290 | rs2531812 | 28532025 | |
| 0291 | rs7382749 | 28533734 | |
| 0292 | rs1041926 | 28534275 | tagSNP #30 |
| 0293 | rs2021745 | 28534965 | |
| 0294 | rs2859372 | 28535109 | tagSNP #31 |
| 0295 | rs2859374 | 28536124 | |
| 0296 | rs9468379 | 28539163 | tagSNP #32 |
| 0297 | rs2859376 | 28541699 | |
| 0298 | rs2531816 | 28550085 | tagSNP #33 |
| 0299 | rs12526477 | 28550942 | |
| 0300 | rs1029328 | 28555894 | |
| 0301 | rs1015811 | 28556065 | |
| 0302 | rs2531817 | 28556873 | |
| 0303 | rs2859350 | 28561132 | |
| 0304 | rs6927023 | 28562200 | |
| 0305 | rs993998 | 28566341 | |
| 0306 | rs13195077 | 28570852 | |
| 0307 | rs6913125 | 28572692 | |
| 0308 | rs2859356 | 28573334 | tagSNP #34 |
| 0309 | rs12661782 | 28574421 | |
| 0310 | rs2191037 | 28574967 | |
| 0311 | rs2531822 | 28576280 | |
| 0312 | rs6937042 | 28576408 | |
| 0313 | rs2071966 | 28581304 | tagSNP #35 |
| 0314 | rs11755403 | 28581907 | |
| 0315 | rs2108925 | 28586203 | |
| 0316 | rs6922986 | 28586574 | |
| 0317 | rs434112 | 28588812 | |
| 0318 | rs406113 | 28591461 | |
| 0319 | rs11757000 | 28592848 | |
| 0320 | rs403774 | 28593258 | |
| 0321 | rs423118 | 28593659 | |
| 0322 | rs9366720 | 28595070 | |
| 0323 | rs2215220 | 28596853 | |
| 0324 | rs377514 | 28599929 | |
| 0325 | rs6924526 | 28604293 | |
| 0326 | rs13202071 | 28605748 | |
| 0327 | rs769189 | 28608214 | |
| 0328 | rs448450 | 28608985 | |
| 0329 | rs1159276 | 28609411 | |
| 0330 | rs426922 | 28610167 | |
| 0331 | rs451774 | 28610529 | tagSNP #36 |
| 0332 | rs413488 | 28611336 | |
| 0333 | rs4533983 | 28613922 | |
| 0334 | rs389118 | 28615358 | |
| 0335 | rs6940168 | 28618957 | |
| 0336 | rs454182 | 28619143 | |
| 0337 | rs2394102 | 28619996 | |
| 0338 | rs384399 | 28621822 | |
| 0339 | rs4711173 | 28622562 | |
| 0340 | rs393414 | 28629295 | tagSNP #37 |
| 0341 | rs6456825 | 28630674 | |
| 0342 | rs414745 | 28634634 | |
| 0343 | rs422089 | 28635385 | |
| 0344 | rs418092 | 28641925 | |
| 0345 | rs454746 | 28645230 | |
| 0346 | rs12193707 | 28650834 | |
| 0347 | rs420463 | 28664033 | |
| 0348 | rs7738979 | 28665291 | tagSNP #38 |
| 0349 | rs418914 | 28676507 | |
| 0350 | rs380914 | 28677928 | |
| 0351 | rs442439 | 28680985 | tagSNP #39 |
| 0352 | rs7773645 | 28682285 | |
| 0353 | rs911178 | 28682394 | |
| 0354 | rs7381750 | 28683907 | |
| 0355 | rs10223644 | 28686951 | |
| 0356 | rs9501180 | 28687450 | |
| 0357 | rs13218450 | 28689302 | |
| 0358 | rs4711176 | 28707007 | |
| 0359 | rs7742658 | 28708471 | |
| 0360 | rs6925972 | 28709913 | |
| 0361 | rs6925044 | 28713410 | |
| 0362 | rs6933018 | 28718340 | tagSNP #40 |
| 0363 | rs963937 | 28718713 | |
| 0364 | rs7773193 | 28719313 | |
| 0365 | rs7743296 | 28722589 | |
| 0366 | rs7382309 | 28728176 | |
| 0367 | rs6910105 | 28731989 | |
| 0368 | rs12210398 | 28734376 | |
| 0369 | rs9295775 | 28735448 | |
| 0370 | rs3051146 | 28735513 | |
| 0371 | rs9885928 | 28758332 | tagSNP #41 |
| 0372 | rs4333411 | 28759712 | |
| 0373 | rs2394123 | 28766679 | |
| 0374 | rs1319076 | 28770369 | |
| 0375 | rs4143771 | 28772610 | |
| 0376 | rs12111360 | 28775427 | |
| 0377 | rs7383248 | 28776459 | |
| 0378 | rs6908726 | 28779322 | |
| 0379 | rs2893974 | 28779877 | |
| 0380 | rs6901325 | 28783609 | |
| 0381 | rs9368571 | 28785737 | |
| 0382 | rs7775835 | 28786336 | tagSNP #42 |
| 0383 | rs1539586 | 28787847 | |
| 0384 | rs6937342 | 28789489 | |
| 0385 | rs9366725 | 28793960 | |
| 0386 | rs5029693 | 28795521 | |
| 0387 | rs6909960 | 28796900 | |
| 0388 | rs1419094 | 28802371 | |
| 0389 | rs2226184 | 28802875 | |
| 0390 | rs1954407 | 28803128 | |
| 0391 | rs7772682 | 28803647 | |
| 0392 | rs9393929 | 28804042 | |
| 0393 | rs12176003 | 28805701 | |
| 0394 | rs7755641 | 28807348 | |
| 0395 | rs10498733 | 28807670 | |
| 0396 | rs6456834 | 28808331 | |
| 0397 | rs7745385 | 28808857 | |
| 0398 | rs6456835 | 28809471 | |
| 0399 | rs7766599 | 28811411 | |
| 0400 | rs2394130 | 28812754 | tagSNP #43 |
| 0401 | rs6905768 | 28813784 | |
| 0402 | rs1233573 | 28814079 | |
| 0403 | rs3869043 | 28814513 | |
| 0404 | rs880638 | 28814890 | |
| 0405 | rs3905240 | 28819312 | |
| 0406 | rs7764322 | 28823296 | |
| 0407 | rs2394128 | 28826851 | |
| 0408 | rs1233590 | 28828699 | |
| 0409 | rs1233591 | 28828969 | |
| 0410 | rs1233596 | 28835668 | |
| 0411 | rs1233597 | 28836324 | |
| 0412 | rs1233599 | 28839178 | |
| 0413 | rs6923801 | 28839751 | |
| 0414 | rs3852213 | 28840354 | |
| 0415 | rs1233603 | 28842021 | |
| 0416 | rs1233606 | 28843503 | |
| 0417 | rs1233610 | 28844978 | |
| 0418 | rs7741972 | 28846980 | |
| 0419 | rs1233616 | 28847874 | |
| 0420 | rs7771281 | 28849271 | |
| 0421 | rs4398727 | 28851341 | |
| 0422 | rs3888926 | 28853266 | |
| 0423 | rs1233619 | 28853442 | |
| 0424 | rs3891338 | 28854516 | |
| 0425 | rs4248135 | 28854839 | |
| 0426 | rs1233621 | 28855641 | |
| 0427 | rs7758798 | 28857010 | tagSNP #44 |
| 0428 | rs7751425 | 28860781 | |
| 0429 | rs7751451 | 28860880 | |
| 0430 | rs1742753 | 28861643 | |
| 0431 | rs4324813 | 28863574 | |
| 0432 | rs12665150 | 28867700 | |
| 0433 | rs878587 | 28869844 | |
| 0434 | rs7745088 | 28872512 | |
| 0435 | rs7454923 | 28874097 | |
| 0436 | rs2765222 | 28875063 | |
| 0437 | rs2765219 | 28878336 | |
| 0438 | rs2754766 | 28878742 | |
| 0439 | rs1967743 | 28879041 | |
| 0440 | rs9368576 | 28880337 | |
| 0441 | rs11758303 | 28880678 | |
| 0442 | rs2754767 | 28883228 | |
| 0443 | rs3131343 | 28883551 | |
| 0444 | rs4324798 | 28884104 | |
| 0445 | rs417919 | 28887410 | |
| 0446 | rs6928657 | 28888296 | |
| 0447 | rs398795 | 28892018 | tagSNP #45 |
| 0448 | rs386628 | 28892383 | |
| 0449 | rs377392 | 28892728 | tagSNP #46 |
| 0450 | rs7762991 | 28893425 | |
| 0451 | rs172330 | 28894871 | |
| 0452 | rs6902254 | 28896033 | |
| 0453 | rs10484543 | 28899189 | |
| 0454 | rs209184 | 28899276 | |
| 0455 | rs209181 | 28900470 | |
| 0456 | rs880157 | 28901772 | |
| 0457 | rs11966708 | 28902266 | |
| 0458 | rs12660128 | 28908373 | |
| 0459 | rs3118357 | 28910142 | |
| 0460 | rs6928422 | 28910791 | |
| 0461 | rs9257248 | 28911285 | |
| 0462 | rs209176 | 28912180 | |
| 0463 | rs422331 | 28918811 | |
| 0464 | rs3131335 | 28922440 | |
| 0465 | rs12180795 | 28930730 | |
| 0466 | rs9501112 | 28932952 | |
| 0467 | rs9380095 | 28933814 | |
| 0468 | rs176461 | 28934399 | tagSNP #47 |
| 0469 | rs9461483 | 28935352 | |
| 0470 | rs3132388 | 28935873 | |
| 0471 | rs209166 | 28937066 | |
| 0472 | rs6456858 | 28938016 | |
| 0473 | rs3132389 | 28939015 | |
| 0474 | rs3131336 | 28939605 | |
| 0475 | rs2187805 | 28940474 | |
| 0476 | rs3118370 | 28941095 | |
| 0477 | rs7775657 | 28943246 | |
| 0478 | rs209164 | 28944450 | |
| 0479 | rs3131093 | 28945431 | |
| 0480 | rs3132392 | 28946623 | |
| 0481 | rs209160 | 28947873 | |
| 0482 | rs7763661 | 28951426 | tagSNP #48 |
| 0483 | rs209153 | 28954243 | |
| 0484 | rs209152 | 28954456 | |
| 0485 | rs209151 | 28954718 | |
| 0486 | rs9501154 | 28956347 | |
| 0487 | rs172326 | 28957784 | |
| 0488 | rs2006758 | 28958376 | |
| 0489 | rs1016472 | 28959361 | |
| 0490 | rs429369 | 28959902 | tagSNP #49 |
| 0491 | rs6930087 | 28960419 | |
| 0492 | rs7750338 | 28961151 | |
| 0493 | rs2032500 | 28962263 | |
| 0494 | rs3135309 | 28963799 | |
| 0495 | rs2148008 | 28963825 | |
| 0496 | rs169679 | 28964566 | |
| 0497 | rs209148 | 28965263 | |
| 0498 | rs209147 | 28966160 | tagSNP #50 |
| 0499 | rs209146 | 28966844 | |
| 0500 | rs7749527 | 28967913 | |
| 0501 | rs6456867 | 28968493 | |
| 0502 | rs381808 | 28970163 | |
| 0503 | rs6942070 | 28971416 | |
| 0504 | rs3130838 | 28974518 | |
| 0505 | rs209131 | 28975745 | |
| 0506 | rs209130 | 28975790 | |
| 0507 | rs1536215 | 28975905 | |
| 0508 | rs209128 | 28977163 | |
| 0509 | rs1056032 | 28978965 | |
| 0510 | rs209126 | 28980436 | |
| 0511 | rs209125 | 28980620 | |
| 0512 | rs209122 | 28983492 | |
| 0513 | rs2269553 | 28984499 | |
| 0514 | rs3135293 | 28985237 | |
| 0515 | rs1794588 | 28985763 | |
| 0516 | rs916284 | 28986621 | |
| 0517 | rs9295781 | 28987764 | |
| 0518 | rs209121 | 28989650 | |
| 0519 | rs3132377 | 28993966 | |
| 0520 | rs9468444 | 28995698 | |
| 0521 | rs6906867 | 28997289 | |
| 0522 | rs9295782 | 28998211 | |
| 0523 | rs2230683 | 28999168 | |
| 0524 | rs1061625 | 28999450 | |
| 0525 | rs2765229 | 29000911 | |
| 0526 | rs2894066 | 29001919 | |
| 0527 | rs3763338 | 29002303 | |
| 0528 | rs1237485 | 29002336 | |
| 0529 | rs12193226 | 29003331 | |
| 0530 | rs2015436 | 29004906 | |
| 0531 | rs1233508 | 29005625 | |
| 0532 | rs3118361 | 29006279 | |
| 0533 | rs2182230 | 29007688 | |
| 0534 | rs932776 | 29009026 | |
| 0535 | rs2032502 | 29009557 | |
| 0536 | rs1004062 | 29010125 | |
| 0537 | rs6912843 | 29012154 | |
| 0538 | rs3135329 | 29013096 | |
| 0539 | rs3130895 | 29013783 | |
| 0540 | rs3131070 | 29014523 | |
| 0541 | rs3130843 | 29016206 | |
| 0542 | rs4713186 | 29017457 | |
| 0543 | rs7762289 | 29018227 | |
| 0544 | rs6931968 | 29018868 | |
| 0545 | rs2071790 | 29019794 | |
| 0546 | rs2071789 | 29020068 | |
| 0547 | rs2071788 | 29020299 | |
| 0548 | rs763009 | 29023100 | |
| 0549 | rs4947339 | 29024244 | |
| 0550 | rs6456876 | 29026928 | |
| 0551 | rs9357074 | 29028080 | |
| 0552 | rs3131073 | 29028964 | |
| 0553 | rs4947256 | 29029932 | |
| 0554 | rs1476016 | 29030711 | |
| 0555 | rs3130845 | 29031359 | tagSNP #51 |
| 0556 | rs9380100 | 29031972 | tagSNP #52 |
| 0557 | rs3131075 | 29032618 | |
| 0558 | rs12386522 | 29036159 | |
| 0559 | rs12110866 | 29046345 | tagSNP #53 |
| 0560 | rs4713190 | 29050823 | |
| 0561 | rs4713191 | 29051859 | |
| 0562 | rs4713192 | 29051870 | |
| 0563 | rs3135322 | 29054910 | |
| 0564 | rs3130837 | 29056082 | |
| 0565 | rs9257445 | 29057196 | |
| 0566 | rs968722 | 29057894 | |
| 0567 | rs9380102 | 29059004 | |
| 0568 | rs6904975 | 29060025 | |
| 0569 | rs12527531 | 29060379 | |
| 0570 | rs3129791 | 29062282 | |
| 0571 | rs3135321 | 29063515 | |
| 0572 | rs3129792 | 29064405 | |
| 0573 | rs6934470 | 29064740 | |
| 0574 | rs6934947 | 29064784 | |
| 0575 | rs10946966 | 29065572 | |
| 0576 | rs7776164 | 29066447 | |
| 0577 | rs6456879 | 29070593 | |
| 0578 | rs2269555 | 29072836 | |
| 0579 | rs6908206 | 29072861 | |
| 0580 | rs9468459 | 29073680 | |
| 0581 | rs6920392 | 29075027 | |
| 0582 | rs6906909 | 29076135 | |
| 0583 | rs9257453 | 29076919 | tagSNP #54 |
| 0584 | rs6933976 | 29077511 | |
| 0585 | rs6456881 | 29078089 | |
| 0586 | rs3129793 | 29078165 | |
| 0587 | rs6456883 | 29078358 | |
| 0588 | rs6916645 | 29082925 | |
| 0589 | rs6923005 | 29084060 | |
| 0590 | rs3130891 | 29085206 | |
| 0591 | rs9468461 | 29087035 | |
| 0592 | rs3130893 | 29088695 | |
| 0593 | rs6930903 | 29089232 | |
| 0594 | rs9357075 | 29090275 | |
| 0595 | rs6916161 | 29091834 | |
| 0596 | rs6916923 | 29092151 | |
| 0597 | rs9468464 | 29092500 | tagSNP #55 |
| 0598 | rs9380103 | 29093193 | |
| 0599 | rs6909302 | 29094363 | |
| 0600 | rs6941946 | 29096571 | |
| 0601 | rs7768299 | 29098183 | |
| 0602 | rs1543796 | 29098606 | |
| 0603 | rs2394512 | 29100172 | |
| 0604 | rs2394513 | 29100496 | |
| 0605 | rs9461497 | 29102115 | |
| 0606 | rs7741520 | 29102558 | |
| 0607 | rs2269554 | 29102977 | |
| 0608 | rs3135320 | 29104537 | |
| 0609 | rs4713197 | 29105573 | |
| 0610 | rs6927986 | 29106722 | |
| 0611 | rs7755402 | 29110050 | |
| 0612 | rs9468471 | 29112079 | |
| 0613 | rs9468473 | 29114238 | |
| 0614 | rs7743837 | 29114832 | |
| 0615 | rs7749435 | 29115835 | tagSNP #56 |
| 0616 | rs6919044 | 29117556 | |
| 0617 | rs6924824 | 29118141 | |
| 0618 | rs6456886 | 29118575 | |
| 0619 | rs6456889 | 29118812 | |
| 0620 | rs7341218 | 29120104 | tagSNP #57 |
| 0621 | rs9468474 | 29121121 | |
| 0622 | rs2143574 | 29122014 | |
| 0623 | rs6456890 | 29123114 | |
| 0624 | rs9380106 | 29123969 | |
| 0625 | rs6456891 | 29124575 | |
| 0626 | rs4713199 | 29125543 | |
| 0627 | rs7747023 | 29133668 | |
| 0628 | rs12665818 | 29140172 | |
| 0629 | rs6904527 | 29141041 | |
| 0630 | rs2179648 | 29144516 | |
| 0631 | rs6918175 | 29147218 | |
| 0632 | rs3131083 | 29147965 | |
| 0633 | rs4713200 | 29148774 | |
| 0634 | rs999265 | 29149791 | |
| 0635 | rs2050231 | 29150377 | |
| 0636 | rs3130758 | 29150925 | |
| 0637 | rs1159519 | 29151568 | tagSNP #58 |
| 0638 | rs3131085 | 29152689 | |
| 0639 | rs9393941 | 29953620 | |
| 0640 | rs2064365 | 29154408 | |
| 0641 | rs9348821 | 29155191 | |
| 0642 | rs3129787 | 29156677 | |
| 0643 | rs11758255 | 29158698 | |
| 0644 | rs7740805 | 29159858 | |
| 0645 | rs6903771 | 29160556 | tagSNP #59 |
| 0646 | rs7752270 | 29161982 | tagSNP #60 |
| 0647 | rs6456901 | 29163426 | |
| 0648 | rs9380109 | 29165955 | |
| 0649 | rs2013972 | 29166981 | |
| 0650 | rs12665108 | 29170178 | |
| 0651 | rs6917293 | 29171042 | |
| D652 | rs4538737 | 29172756 | |
| 0653 | rs6907184 | 29173378 | |
| 0654 | rs4713201 | 29175247 | |
| 0655 | rs6905729 | 29176613 | |
| 0656 | rs2394518 | 29177328 | |
| 0657 | rs9295790 | 29178419 | |
| 0658 | rs3131090 | 29180277 | |
| 0659 | rs3131091 | 29181083 | |
| 0660 | rs6456908 | 29183061 | |
| 0661 | rs11754944 | 29183651 | |
| 0662 | rs3130762 | 29185776 | |
| 0663 | rs3116838 | 29186140 | |
| 0664 | rs6456909 | 29187145 | |
| 0665 | rs3749977 | 29188333 | |
| 0666 | rs3130764 | 29188338 | |
| 0667 | rs3129106 | 29189150 | |
| 0668 | rs3129109 | 29192219 | |
| 0669 | rs3129110 | 29193215 | |
| 0670 | rs3130766 | 29194816 | |
| 0671 | rs2064162 | 29196802 | |
| 0672 | rs7740128 | 29202683 | |
| 0673 | rs6456913 3 | 29204982 | |
| 0674 | rs7760364 | 29205923 | |
| 0675 | rs5930603 | 29206817 | |
| 0676 | rs3130778 | 29207566 | |
| 0677 | rs7738990 | 29209108 | |
| 0678 | rs3129119 | 29210847 | |
| 0679 | rs12529022 | 29211556 | |
| 0680 | rs2223363 | 29212656 | |
| 0681 | rs9295794 | 29212919 | |
| 0682 | rs7775256 | 29213780 | |
| 0683 | rs2267632 | 29214452 | |
| 0684 | rs9488487 | 29215036 | |
| 0685 | rs9468488 | 29215616 | |
| 0686 | rs9393945 | 29216275 | |
| 0687 | rs6917665 | 29217585 | |
| 0688 | rs3116846 | 29222605 | |
| 0689 | rs3130718 | 29222610 | |
| 0690 | rs3130720 | 29223737 | |
| 0691 | rs3130724 | 29226518 | |
| 0692 | rs6902241 | 29227147 | |
| 0693 | rs3129126 | 29229623 | |
| 0694 | rs7750858 | 29234658 | |
| 0695 | rs3130732 | 29235461 | |
| 0696 | rs2142906 | 29237975 | |
| 0697 | rs3130737 | 29243200 | |
| 0698 | rs3918428 | 29244102 | |
| 0699 | rs2394520 | 29245212 | |
| 0700 | rs3129151 | 29246307 | tagSNP #61 |
| 0701 | rs3129154 | 29247778 | |
| 0702 | rs9468491 | 29248729 | |
| 0703 | rs3116855 | 29249624 | |
| 0704 | rs3129157 | 29249735 | |
| 0705 | rs3129158 | 29249764 | |
| 0706 | rs3116856 | 29249841 | |
| 0707 | rs3129159 | 29250046 | |
| 0708 | rs3130743 | 29250056 | |
| 0709 | rs2206042 | 29250468 | |
| 0710 | rs3116857 | 29252119 | |
| 0711 | rs3130745 | 29256611 | |
| 0712 | rs3116817 | 29257553 | |
| 0713 | rs2006752 | 29259304 | |
| 0714 | rs3116820 | 29259663 | |
| 0715 | rs3129171 | 29263745 | |
| 0716 | rs3129173 | 29267640 | |
| 0717 | rs6904810 | 29269451 | |
| 0718 | rs1977074 | 29271850 | tagSNP #62 |
| 0719 | rs3129093 | 29278281 | |
| 0720 | rs760804 | 29279059 | |
| 0721 | rs6906270 | 29279872 | tagSNP #63 |
| 0722 | rs2394546 | 29283619 | |
| 0723 | rs736466 | 29284316 | |
| 0724 | rs1883329 | 29285120 | tagSNP #64 |
| 0725 | rs2206040 | 29286532 | |
| 0726 | rs2206041 | 29286624 | |
| 0727 | rs3130817 | 29287255 | |
| 0728 | rs3129095 | 29288303 | |
| 0729 | rs719746 | 29289617 | |
| 0730 | rs2894083 | 29290325 | |
| 0731 | rs3129096 | 29291365 | |
| 0732 | rs11758554 | 29291888 | |
| 0733 | rs3116837 | 29292679 | |
| 0734 | rs6933230 | 29293306 | tagSNP #65 |
| 0735 | rs3130801 | 29296198 | tagSNP #66 |
| 0736 | rs3130803 | 29297174 | |
| 0737 | rs714470 | 29299430 | |
| 0738 | rs7757500 | 29300165 | |
| 0739 | rs3130811 | 29301289 | |
| 0740 | rs3130812 | 29301804 | |
| 0741 | rs3130813 | 29303030 | |
| 0742 | rs6901837 | 29304156 | |
| 0743 | rs3130814 | 29304200 | |
| 0744 | rs2207337 | 29308264 | |
| 0745 | rs10456369 | 29309134 | |
| 0746 | rs3117345 | 29310484 | |
| 0747 | rs3130816 | 29311930 | |
| 0748 | rs7742939 | 29313352 | |
| 0749 | rs7770244 | 29320306 | |
| 0750 | rs3129105 | 29321059 | |
| 0751 | rs7747464 | 29322936 | |
| 0752 | rs3117330 | 29333788 | |
| 0753 | rs3117329 | 29335636 | tagSNP #67 |
| 0754 | rs7761697 | 29338093 | |
| 0755 | rs3117328 | 29338570 | |
| 0756 | rs3130827 | 29338676 | |
| 0757 | rs10484545 | 29342503 | |
| 0758 | rs6904130 | 29343186 | |
| 0759 | rs3130829 | 29343893 | |
| 0760 | rs6942318 | 29346010 | |
| 0761 | rs3117327 | 29347145 | |
| 0762 | rs3117326 | 29348372 | |
| 0763 | rs2394550 | 29356453 | |
| 0764 | rs5875188 | 29356556 | tagSNP #68 |
| 0765 | rs6456942 | 29359392 | |
| 0766 | rs9468508 | 29361748 | |
| 0767 | rs6901923 | 29361879 | |
| 0768 | rs7741086 | 29362358 | |
| 0769 | rs1884123 | 29364431 | tagSNP #69 |
| 0770 | rs1033569 | 29365783 | |
| 0771 | rs1033568 | 29365978 | |
| 0772 | rs3130835 | 29366874 | |
| 0773 | rs4446587 | 29367634 | |
| 0774 | rs3117425 | 29368442 | |
| 0775 | rs4398768 | 29369046 | |
| 0776 | rs7383161 | 29372666 | |
| 0777 | rs6899932 | 29374102 | tagSNP #70 |
| 0778 | rs7754926 | 29377077 | |
| 0779 | rs3117424 | 29377294 | |
| 0780 | rs4594993 | 29378508 | |
| 0781 | rs3117426 | 29380022 | |
| 0782 | rs7774255 | 29382366 | |
| 0783 | rs9257694 | 29382496 | |
| 0784 | rs1474859 | 29383595 | |
| 0785 | rs9257697 | 29384190 | |
| 0786 | rs6910451 | 29388164 | |
| 0787 | rs9393954 | 29390362 | |
| 0788 | rs9257711 | 29391455 | |
| 0789 | rs1535151 | 29392363 | |
| 0790 | rs720831 | 29392530 | |
| 0791 | rs9257712 | 29393201 | tagSNP #71 |
| 0792 | rs4713209 | 29395301 | |
| 0793 | rs9257718 | 29399739 | |
| 0794 | rs9468515 | 29400272 | |
| 0795 | rs9257723 | 29403613 | |
| 0796 | rs12660111 | 29404160 | tagSNP #72 |
| 0797 | rs9257726 | 29404935 | |
| 0798 | rs9357078 | 29405678 | |
| 0799 | rs9257731 | 29407383 | |
| 0800 | rs6930435 | 29409234 | tagSNP #73 |
| 0801 | rs6903989 | 29410042 | |
| 0802 | rs6917520 | 29412559 | |
| 0803 | rs6927977 | 29413965 | |
| 0804 | rs3130805 | 29414468 | |
| 0805 | rs9348827 | 29416405 | |
| 0806 | rs9257748 | 29417780 | |
| 0807 | rs1014258 | 29418692 | |
| 0808 | rs9257751 | 29419450 | |
| 0809 | rs3117435 | 29420773 | |
| 0810 | rs7745768 | 29422975 | tagSNP #74 |
| 0811 | rs9257756 | 29424211 | |
| 0812 | rs4321865 | 29424902 | |
| 0813 | rs9257768 | 29430097 | tagSNP #75 |
| 0814 | rs7356951 | 29430664 | |
| 0815 | rs9257769 | 29431087 | |
| 0816 | rs3117438 | 29431270 | |
| 0817 | rs9257770 | 29431849 | |
| 0818 | rs6930033 | 29431916 | |
| 0819 | rs7357041 | 29433110 | |
| 0820 | rs4713210 | 29433444 | |
| 0821 | rs3129685 | 29433613 | |
| 0822 | rs9257777 | 29435169 | |
| 0823 | rs7754402 | 29436845 | |
| 0824 | rs16718 | 29443137 | |
| 0825 | rs9380120 | 29443563 | |
| 0826 | rs4713211 | 29444080 | |
| 0827 | rs9295804 | 29444173 | |
| 0828 | rs6934993 | 29445526 | |
| 0829 | rs4713213 | 29446988 | |
| 0830 | rs7772982 | 29449033 | |
| 0831 | rs7753474 | 29449334 | tagSNP #76 |
| 0832 | rs7773534 | 29449390 | |
| 0833 | rs9380122 | 29450262 | |
| 0834 | rs3749971 | 29450801 | |
| 0835 | rs3749970 | 29450851 | |
| 0836 | rs5003264 | 29452015 | |
| 0837 | rs2144425 | 29452965 | |
| 0838 | rs2144426 | 29453055 | |
| 0839 | rs238884 | 29453703 | |
| 0840 | rs238880 | 29455070 | |
| 0841 | rs1419640 | 29458879 | tagSNP #77 |
| 0842 | rs238872 | 29459898 | |
| 0843 | rs3117444 | 29460965 | |
| 0844 | rs3094555 | 29461678 | |
| 0845 | rs3129681 | 29462403 | |
| 0846 | rs3094549 | 29463168 | |
| 0847 | rs1419638 | 29463539 | |
| 0848 | rs1419637 | 29463578 | |
| 0849 | rs1419635 | 29463938 | |
| 0850 | rs4711185 | 29464066 | |
| 0851 | rs442694 | 29464707 | |
| 0852 | rs925781.3 | 29465382 | |
| 0853 | rs4713214 | 29466016 | |
| 0854 | rs7452887 | 29466696 | |
| 0855 | rs4452630 | 29466953 | |
| 0856 | rs9257819 | 29468203 | |
| 0857 | rs1362065 | 29468697 | |
| 0858 | rs2022077 | 29469144 | |
| 0859 | rs9257823 | 29469755 | |
| 0860 | rs1362063 | 29470313 | |
| 0861 | rs9257827 | 29470923 | tagSNP #78 |
| 0862 | rs4713216 | 29472024 | |
| 0863 | rs4713217 | 29472202 | |
| 0864 | rs9257834 | 29472492 | |
| 0865 | rs3128853 | 29472664 | |
| 0866 | rs2073154 | 29472692 | |
| 0867 | rs2073151 | 29472828 | |
| 0868 | rs2073150 | 29473118 | |
| 0869 | rs2073149 | 29473300 | |
| 0870 | rs4713218 | 29473851 | |
| 0871 | rs2158279 | 29474142 | |
| 0872 | rs1024470 | 29474254 | |
| 0873 | rs1028411 | 29475276 | |
| 0874 | rs4713220 | 29475375 | |
| 0875 | rs4711187 | 29475467 | |
| 0876 | rs4713221 | 29475619 | |
| 0877 | rs1819784 | 29476152 | |
| 0878 | rs9405124 | 29476682 | |
| 0879 | rs2394604 | 29477135 | |
| 0880 | rs2394605 | 29477187 | |
| 0881 | rs2394606 | 29477219 | |
| 0882 | rs2394607 | 29477386 | |
| 0883 | rs1419634 | 29477539 | |
| 0884 | rs2894099 | 29478724 | |
| 0885 | rs1362061 | 29479032 | |
| 0886 | rs1362050 | 29479282 | |
| 0887 | rs429479 | 29480190 | |
| 0888 | rs994321 | 29480223 | |
| 0889 | rs1419633 | 29481080 | |
| 0890 | rs1419632 | 29481116 | |
| 0891 | rs7453752 | 29484933 | |
| 0892 | rs2097771 | 29486306 | |
| 0893 | rs12192194 | 29487758 | |
| 0894 | rs1544403 | 29489773 | tagSNP #79 |
| 0895 | rs720497 | 29490300 | tagSNP #80 |
| 0896 | rs1362074 | 29491126 | |
| 0897 | rs1362073 | 29491187 | |
| 0898 | rs3029487 | 29491488 | |
| 0899 | rs10807055 | 29491998 | |
| 0900 | rs3131024 | 29493104 | |
| 0901 | rs1011985 | 29493639 | |
| 0902 | rs12207410 | 29494473 | |
| 0903 | rs6456947 | 29496173 | |
| 0904 | rs2523421 : | 29498307 | |
| 0905 | rs1419643 | 29499801 | |
| 0906 | rs7754054 | 29500368 | |
| 0907 | rs2074470 | 29502292 | |
| 0908 | rs7770592 | 29503612 | tagSNP #81 |
| 0909 | rs7757269 | 29504082 | |
| 0910 | rs10946990 | 29504511 | |
| 0911 | rs6904456 | 29505417 | |
| 0912 | rs2394609 | 29505865 | |
| 0913 | rs6910208 | 29505948 | |
| 0914 | rs3131025 | 29506680 | tagSNP #82 |
| 0915 | rs7349863 | 29506724 | |
| 0916 | rs11754009 | 29514561 | |
| 0917 | rs9468532 | 29515243 | |
| 0918 | rs2074469 | 29515835 | |
| 0919 | s2074466 | 29516178 | |
| 0920 | rs9468533 | 29516740 | |
| 0921 | rs7765791 | 29517958 | tagSNP #83 |
| 0922 | rs11752013 | 29518624 | |
| 0923 | rs6937718 | 29519614 | |
| 0924 | rs9368601 | 29520288 | |
| 0925 | rs7738722 | 29522151 | |
| 0926 | rs7738742 | 29522211 | |
| 0927 | rs7739243 | 29522478 | |
| 0928 | rs6929603 | 29523517 | |
| 0929 | rs6935708 | 29524519 | |
| 0930 | rs2523442 | 29525684 | |
| 0931 | rs3094574 | 29529959 | |
| 0932 | rs1419647 | 29530551 | |
| 0933 | rs9257863 | 29533453 | |
| 0934 | rs6456951 | 29534072 | |
| 0935 | rs6903755 | 29534858 | |
| 0936 | rs7768854 | 29536699 | |
| 0937 | rs2021729 | 29537260 | |
| 0938 | rs2073148 | 29538038 | |
| 0939 | rs3128854 | 29539566 | |
| 0940 | rs1345228 | 29540258 | tagSNP #84 |
| 0941 | rs2107191 | 29542163 | |
| 0942 | rs2746149 | 29543223 | |
| 0943 | rs1419646 | 29544310 | |
| 0944 | rs6937864 | 29545795 | |
| 0945 | rs11961170 | 29546973 | |
| 0946 | rs11966831 | 29548230 | |
| 0947 | rs7751705 | 29549486 | |
| 0948 | rs2746150 | 29550569 | |
| 0949 | rs1233495 | 29551989 | |
| 0950 | rs7756110 | 29553363 | |
| 0951 | rs6925408 | 29554438 | |
| 0952 | rs3094573 | 29554500 | tagSNP #85 |
| 0953 | rs6925744 | 29554557 | |
| 0954 | rs7771335 | 29555906 | |
| 0955 | rs2107189 | 29559036 | |
| 0956 | rs11756628 | 29559867 | |
| 0957 | rs10484547 | 29560642 | |
| 0958 | rs9501675 | 29561168 | |
| 0959 | rs11965733 | 29562018 | |
| 0960 | rs2066951 | 29562357 | |
| 0961 | rs9348832 | 29563889 | |
| 0962 | rs11756938 | 29564522 | |
| 0963 | rs1233493 | 29566109 | |
| 0964 | rs1233492 | 29566345 | |
| 0965 | rs3128844 | 29567128 | |
| 0966 | rs1233491 | 29569598 | |
| 0967 | rs1233490 | 29569782 | |
| 0968 | rs6915084 | 29570049 | |
| 0969 | rs1233489 | 29570911 | |
| 0970 | rs6926506 | 29571836 | |
| 0971 | rs6932526 | 29572837 | |
| 0972 | rs1345227 | 29574824 | |
| 0973 | rs7766082 | 29575874 | |
| 0974 | rs1233487 | 29576677 | tagSNP #86 |
| 0975 | rs9393967 | 29577357 | |
| 0976 | rs1233486 | 29577520 | |
| 0977 | rs4711192 | 29578654 | |
| 0978 | rs3131019 | 29578853 | |
| 0979 | rs11961013 | 29579802 | |
| 0980 | rs757256 | 29580784 | |
| 0981 | rs3130858 | 29581369 | tagSNP #87 |
| 0982 | rs3130860 | 29582253 | |
| 0983 | rs734960 | 29583010 | |
| 0984 | rs1002187 | 29583335 | |
| 0985 | rs1233482 | 29583550 | |
| 0986 | rs3131020 | 29583770 | |
| 0987 | rs3131021 | 29584010 | |
| 0988 | rs3131022 | 29584552 | |
| 0989 | rs1557820 | 29585108 | |
| 0990 | rs2158281 | 29585651 | |
| 0991 | rs1233478. | 29585689 | |
| 0992 | rs2285791 | 29586169 | |
| 0993 | rs1010408 | 29587401 | tagSNP #88 |
| 0994 | rs12527641 | 29588293 | |
| 0995 | rs3094572 | 29588861 | |
| 0996 | rs1362075 | 29589996 | |
| 0997 | rs2523433 | 29590300 | |
| 0998 | rs1592410 | 29591842 | |
| 0999 | rs1015869 | 29592541 | |
| 1000 | rs1015868 | 29593248 | |
| 1001 | rs3094564 | 29594220 | |
| 1002 | rs9257890 | 29594783 | |
| 1003 | rs2745400 | 29596124 | |
| 1004 | rs724078 | 29596927 | |
| 1005 | rs7752486 | 29600442 | |
| 1006 | rs2745403 | 29601050 | |
| 1007 | rs7450360 | 29601862 | |
| 1008 | rs969931 | 29602775 | |
| 1009 | rs1233427 | 29604542 | |
| 1010 | rs9468549 | 29605447 | |
| 1011 | rs1233426 | 29605980 | |
| 1012 | rs6908631 | 29606301 | |
| 1013 | rs1233425 | 29606375 | |
| 1014 | rs362546 | 29607294 | |
| 1015 | rs909967 | 29607415 | |
| 1015 | rs886381 | 29608103 | |
| 1017 | rs362544 | 29608165 | |
| 1018 | rs1233422 | 29608290 | |
| 1019 | rs9968 | 29608915 | |
| 1020 | rs2294748 | 29609060 | |
| 1021 | rs1233421 | 29609392 | tagSNP #89 |
| 1022 | rs1233420 | 29611201 | |
| 1023 | rs6930217 | 29611660 | |
| 1024 | rs6911709 | 29611821 | |
| 1025 | rs6911894 | 29611890 | |
| 1026 | rs6912673 | 29612037 | |
| 1027 | rs6935418 | 29612135 | |
| 1028 | rs1233418 | 29613022 | |
| 1029 | rs419957 | 29613647 | |
| 1030 | rs414282 | 29614138 | |
| 1031 | rs422241 | 29614178 | |
| 1032 | rs362514 | 29615188 | |
| 1033 | rs6936699 | 29616108 | |
| 1034 | rs10484548 | 29616151 | |
| 1035 | rs414390 | 29617752 | |
| 1036 | rs362541 | 29618128 | |
| 1037 | rs362540 | 29618203 | |
| 1038 | rs3025657 | 29619094 | |
| 1039 | rs2534795 | 29619219 | |
| 1040 | rs6935895 | 29619639 | |
| 1041 | rs2534794 | 29619728 | tagSNP #90 |
| 1042 | rs407161 | 29621698 | |
| 1043 | rs3094576 | 29624135 | |
| 1044 | rs376681 | 29624986 | |
| 1045 | rs365488 | 29625851 | |
| 1046 | rs2745411 | 29626338 | |
| 1047 | rs417374 | 29626368 | |
| 1048 | rs7763501 | 29626830 | |
| 1049 | rs5875195 | 29627328 | |
| 1050 | rs3052069 | 29627329 | |
| 1051 | rs3906305 | 29627455 | |
| 1052 | rs453658 | 29627744 | |
| 1053 | rs446145 | 29628683 | |
| 1054 | rs11724 | 29629215 | |
| 1055 | rs389419 | 29629341 | |
| 1056 | rs7771629 | 29629845 | |
| 1057 | rs3215532 | 29630014 | tagSNP #91 |
| 1058 | rs2294745 | 29630048 | |
| 1059 | rs6915177 | 29630484 | |
| 1060 | rs2534791 | 29630608 | |
| 1061 | rs7739536 | 29631368 | |
| 1062 | rs444013 | 29631542 | |
| 1063 | rs8337 | 29631572 | |
| 1064 | rs404240 | 29631853 | tagSNP #92 |
| 1065 | rs2076484 | 29631899 | |
| 1066 | rs2534790 | 29632064 | |
| 1067 | rs12665228 | 29633487 | |
| 1068 | rs362536 | 29634836 | |
| 1069 | rs64036 | 29635163 | |
| 1070 | rs2272991 | 29635495 | |
| 1071 | rs362513 | 29636214 | |
| 1072 | rs995185 | 29637033 | |
| 1073 | rs1233405 | 29637650 | |
| 1074 | rs362531 | 29638351 | |
| 1075 | rs362512 | 29638524 | |
| 1076 | rs422878 | 29638840 | |
| 1077 | rs362527 | 29639714 | |
| 1078 | rs2523447 | 29640631 | tagSNP #93 |
| 1079 | rs388234 | 29641187 | |
| 1080 | rs3094577 | 29641762 | |
| 1081 | rs1119080 | 29644577 | |
| 1082 | rs1235162 | 29645116 | |
| 1083 | rs1003582 | 29646295 | |
| 1084 | rs12527115 | 29647093 | |
| 1085 | rs1233399 | 29647374 | |
| 1086 | rs1003581 | 29648096 | tagSNP #94 |
| 1087 | rs362509 | 29648753 | tagSNP #95 |
| 1088 | rs6912437 | 29649286 | |
| 1089 | rs362525 | 29651538 | |
| 1090 | rs362524 | 29651665 | |
| 1091 | rs362522 | 29653100 | tagSNP #96 |
| 1092 | rs1233397 | 29653607 | tagSNP #97 |
| 1093 | rs4568477 | 29654804 | |
| 1094 | rs1233391 | 29658510 | |
| 1095 | rs1233389 | 29660375 | |
| 1096 | rs1233388 | 29661819 | |
| 1097 | rs3129034 | 29663701 | |
| 1098 | rs362521 | 29664651 | tagSNP #98 |
| 1099 | rs1233386 | 29666082 | |
| 1100 | rs1233384 | 29667187 | tagSNP #99 |
| 1101 | rs6912589 | 29669742 | |
| 1102 | rs3095273 | 29674261 | |
| 1103 | rs3025644 | 29675815 | |
| 1104 | rs3025643 | 29677847 | tagSNP #100 |
| 1105 | rs2267633 | 29678733 | |
| 1106 | rs2076483 | 29679437 | tagSNP #101 |
| 1107 | rs10946999 | 29679501 | |
| 1108 | rs3025638 | 29680813 | |
| 1109 | rs2076482 | 29681146 | |
| 1110 | rs12193396 | 29682289 | |
| 1111 | rs5875197 | 29682488 | |
| 1112 | rs740884 | 29682523 | |
| 1113 | rs740882 | 29683348 | |
| 1114 | rs881284 | 29683939 | |
| 1115 | rs29230 | 29684285 | tagSNP #102 |
| 1116 | rs2076489 | 29684329 | |
| 1117 | rs29263 | 29684482 | |
| 1118 | rs29262 | 29684484 | |
| 1119 | rs29258 | 29685509 | |
| 1120 | rs29257 | 29685922 | |
| 1121 | rs2076488 | 29686421 | |
| 1122 | rs29255 | 29687436 | |
| 1123 | rs29253 | 29688328 | |
| 1124 | rs29227 | 29688480 | |
| 1125 | rs29226 | 29688903 | tagSNP #103 |
| 1126 | rs29225 | 29688933 | |
| 1127 | rs29251 | 29689649 | |
| 1128 | rs6927867 | 29690417 | |
| 1129 | rs6919973 | 29695767 | |
| 1130 | rs29223 | 29696437 | |
| 1131 | rs25629 | 29697294 | |
| 1132 | rs29220 | 29697559 | |
| 1133 | rs3828923 | 29898608 | |
| 1134 | rs6938190 | 29698760 | |
| 1135 | rs6938734 | 29698952 | |
| 1136 | rs3025626 | 29699757 | |
| 1137 | rs2267635 | 29700324 | |
| 1138 | rs715044 | 29701681 | |
| 1139 | rs2021749 | 29706012 | |
| 1140 | rs29243 | 29706995 | |
| 1141 | rs9257924 | 29711023 | |
| 1142 | rs9461540 | 29712152 | |
| 1143 | rs444189 | 29713823 | |
| 1144 | rs3095267 | 29714937 | tagSNP #104 |
| 1145 | rs3025623 | 29715638 | |
| 1146 | rs1233374 | 29716587 | |
| 1147 | rs29236 | 29717102 | |
| 1148 | rs3117289 | 29717814 | |
| 1149 | rs29273 | 29718882 | tagSNP #1D5 |
| 1150 | rs29233 | 29719122 | |
| 1151 | rs29232 | 29719324 | |
| 1152 | rs3131854 | 29719778 | tagSNP #106 |
| 1153 | rs3129073 | 29723708 | |
| 1154 | rs439812 | 29724493 | tagSNP #107 |
| 1155 | rs29269 | 29725632 | |
| 1158 | rs29272 | 29726251 | |
| 1157 | rs29228 | 29731622 | |
| 1158 | rs29234 | 29731995 | tagSNP #108 |
| 1159 | rs3130250 | 29732884 | |
| 1160 | rs2535267 | 29733641 | |
| 1161 | rs2252711 | 29734203 | |
| 1162 | rs2535260 | 29736767 | |
| 1163 | rs2256266 | 29740203 | |
| 1164 | rs6905408 | 29741707 | tagSNP #109 |
| 1165 | rs2071652 | 29743249 | |
| 1166 | rs2071653 | 29743860 | |
| 1167 | rs2273192 | 29746111 | |
| 1168 | rs9357083 | 29747576 | |
| 1169 | rs2535242 | 29748614 | tagSNP #110 |
| 1170 | rs9461544 | 29749235 | |

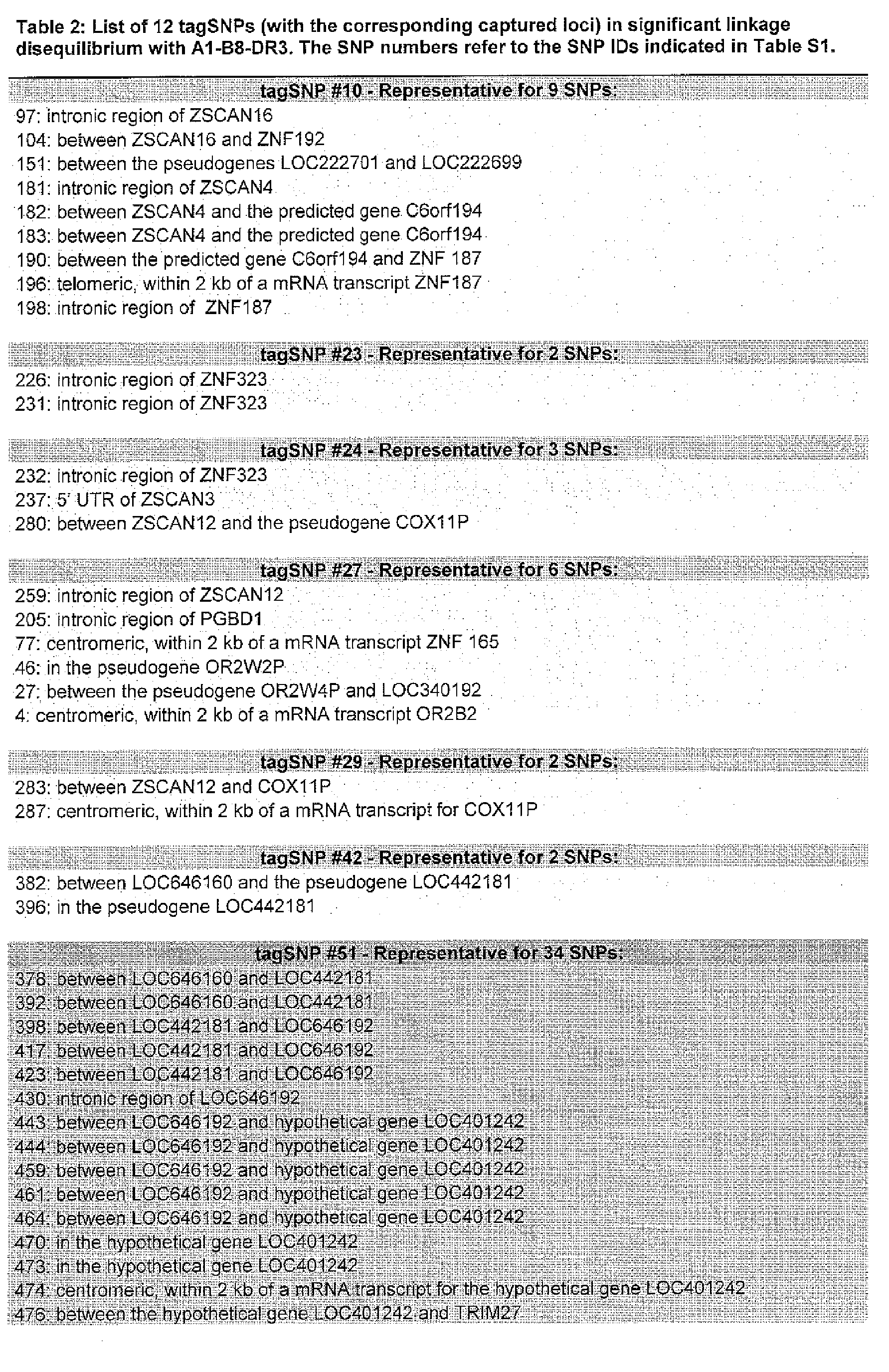

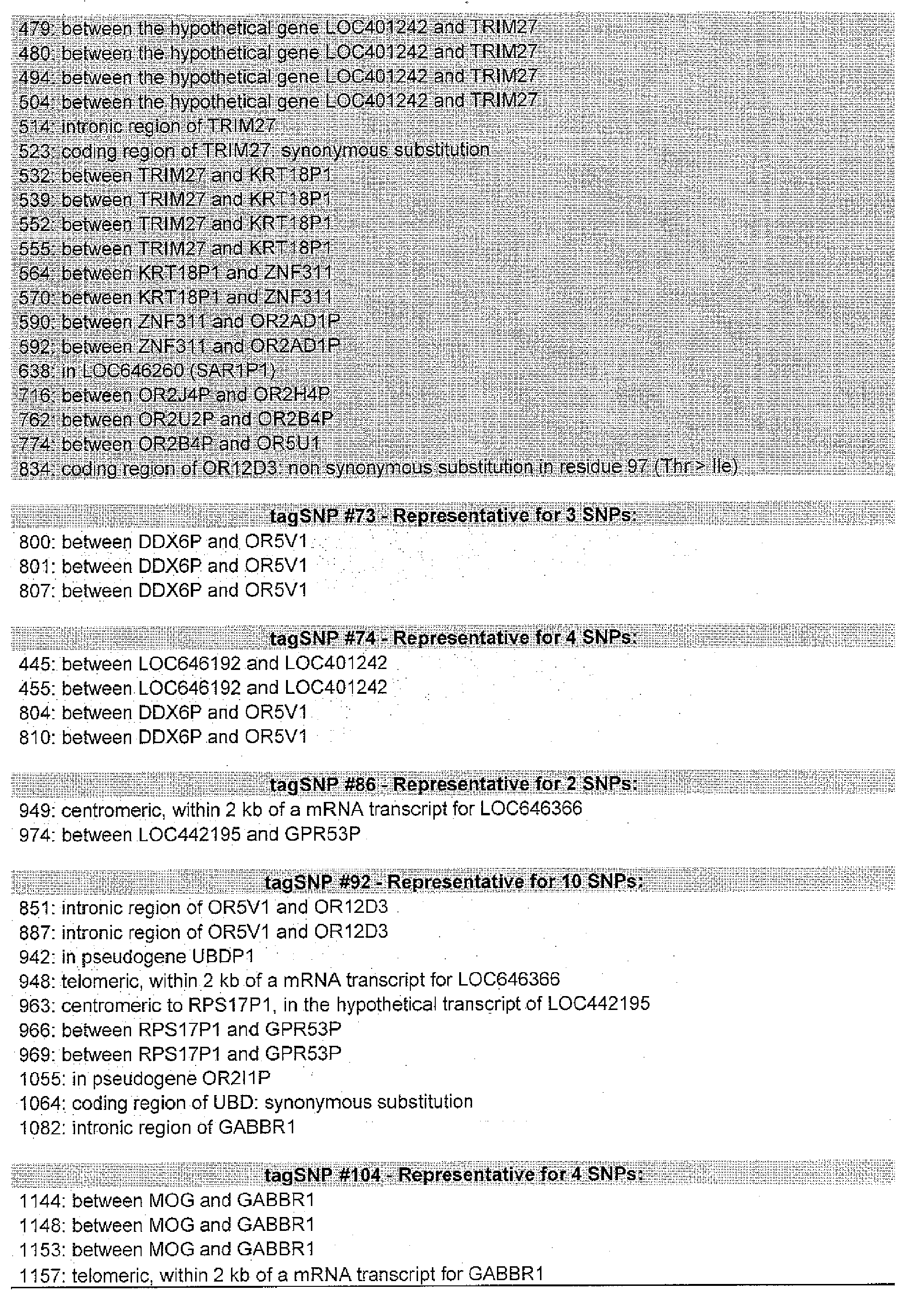

**Table 3: Compilation of SNPs determining non-synonymous exchanges between OR2B2 and MOG. 21 coding, non-synonymous SNPs from this genomic region with the corresponding dbSNP lDs, NCB136 coordinates, alleles, gene designations, the domain they occur in (in case of OR), position in the protein, and amino acid substitution that they may cause. IC1-3: intracellular domains one to three; EC1-3: extracellular domains one to three; TM1-7: transmembrane domains one to seven; N-ter: N terminus; * the position of the amino acid within a particular domain is conflicting between different prediction algorithms.**

| | | | | | | | |
|---|---|---|---|---|---|---|---|
| **SNP order in** **figure 2B** | **dbSNP ID** | **Coordinate (NCBI 36)** | **Alleles** | **Locus** | **Domain in receptor** | **Amino acid position** | **Amino acid change** |
| 1 | rs6456811 | 28378026 | C/G | PGBD1 | - | 806 | H-D |
| 2 | rs406113 | 28591461 | A/C | GPX6 | - | 13 | F-L |
| 3 | rs3749977 | 29188323 | C/T | OR2J3 | IC3 | 226 | R-Q |
| 4 | rs3130764 | 29188328 | A/G | OR2J3 | IC3 | 228 | I-V |
| 5 | rs3116855 | 29249611 | C/T | OR2J2 | TM2 | 74 | H-Y |
| 6 | rs3129157 | 29249722 | A/G | OR2J2 | TM3 | 111 | T-A |
| 7 | rs3116856 | 29249828 | C/T | OR2J2 | TM4 | 146 | A-V |
| 8 | rs3130743 | 29250043 | A/G | OR2J2 | TM5 | 218 | T-A |
| 9 | rs9257694 | 29382465 | C/T | OR5U1 | N-ter | 7 | T-M |
| 10 | rs9257770 | 29431817 | A/C | OR5V1 | TM1 | 45 | I-M |
| 11 | rs6930033 | 29431884 | A/C | OR5V1 | N-ter | 23 | L-W |
| 12 | rs3749971 | 29450754 | C/T | OR12D3 | EC1 or TM3* | 97 | T-1 |
| 13 | rs9257834 | 29472594 | G/T | OR12D2 | TM1 or IC1* | 47 | V-F |
| 14 | rs3128853 | 29472766 | C/T | OR12D2 | EC1 or TM3* | 104 | S-F |
| 15 | rs2073154 | 29472794 | C/G | OR12D2 | TM3 | 113 | F-L |
| 16 | rs2073151 | 29472930 | C/T | OR12D2 | TM4 or EC2* | 159 | V-I |
| 17 | rs2074469 | 29515949 | A/G | OR10C1 | TM2 | 60 | F-L |
| 18 | rs2074466 | 29516292 | G/T | OR10C1 | EC2 | 174 | P-Q |
| 19 | rs2076484 | 29631982 | C/T | UBD | - | 51 | S-L |
| 20 | rs3129034 | 29663788 | A/C/G/T | OR2H2 | TM1 | 30 | I-L-V-F |
| 21 | rs2076489 | 29684416 | C/T | GABBR1 | - | 645/528/583 | L-F |

## Claims

1. A method for in-vitro diagnosing a predisposition to smoking comprising the steps of:
- incubating a sample of a body fluid taken from a human with substances being specific to at least one single nucleotide polymorphism (SNP) that is selected from the group comprising the SNPs rs6908726, rs9393929, rs6456835, rs1233610, rs1233619, rs1742753, rs3131343, rs4324798, rs3118357, rs9257248, rs3131335, rs3132388, rs3132389, rs3131336, rs3118370, rs3131093, rs3132392, rs3135309, rs3130838, rs3135293, rs2230683, rs3118361, rs3130895, rs3131073, rs3130845, rs3130837, rs3129791, rs3130891, rs3130893, rs3131085, rs3129173, rs3117326, rs3117425 and rs3749971, or at least one single amino acid polymorphism (SAAP) that is encoded by any of said SNPs, and
- detecting the specific incubation products.

2. Method according to claim 1, wherein the SNP rs3749971 within the OR12D3 gene or the SAAP specifying isoleucine at position 97 within the OR12D3 protein is selected.

3. Method according to claim 1 or 2, wherein the incubating step comprises hybridizing primers with the OR12D3 gene and amplifying the region between them, preferably hybridizing primers that comprise at least one DNA sequence having the SEQ ID NOs: 1 to 3.

4. Pharmaceutical composition comprising at least one substance specifically interacting with the OR12D3^{97Ile} protein having the SEQ ID NO: 4, optionally together with pharmaceutically tolerable adjuvants.

5. Pharmaceutical composition according to claim 4, wherein the substance is an antagonist and/or a molecule that mimics the ligand binding of a bergamot oil ingredient to the OR12D3^{97Ile} protein, preferably the binding of a monoterpene, more preferably the binding of linalool.

6. Use of substances specifically interacting with the OR12D3^{97Ile} protein having the SEQ ID NO: 4 for the production of a medicament for the prophylactic or therapeutic treatment of tobacco abuse.

7. Use of substances being specific to at least one single nucleotide polymorphism (SNP) that is selected from the group comprising the SNPs rs6908726, rs9393929, rs6456835, rs1233610, rs1233619, rs1742753, rs3131343, rs4324798, rs3118357, rs9257248, rs3131335, rs3132388, rs3132389, rs3131336, rs3118370, rs3131093, rs3132392, rs3135309, rs3130838, rs3135293, rs2230683, rs3118361, rs3130895, rs3131073, rs3130845, rs3130837, rs3129791, rs3130891, rs3130893, rs3131085, rs3129173, rs3117326, rs3117425 and rs3749971, or at least one single amino acid polymorphism (SAAP) that is encoded by any of said SNPs, for in-vitro diagnosis of a predisposition to smoking.

8. Kit for use in diagnosis of a predisposition to smoking comprising substances being specific to at least one single nucleotide polymorphism (SNP) that is selected from the group comprising the SNPs rs6908726, rs9393929, rs6456835, rs1233610, rs1233619, rs1742753, rs3131343, rs4324798, rs3118357, rs9257248, rs3131335, rs3132388, rs3132389, rs3131336, rs3118370, rs3131093, rs3132392, rs3135309, rs3130838, rs3135293, rs2230683, rs3118361, rs3130895, rs3131073, rs3130845, rs3130837, rs3129791, rs3130891, rs3130893, rs3131085, rs3129173, rs3117326, rs3117425 and rs3749971, or at least one single amino acid polymorphism (SAAP) that is encoded by any of said SNPs.

9. A method for screening substances, which reduce the predilection for ingredients of tobacco, comprising the steps of:
- providing at least two subjects of a non-human organism being capable of expressing the OR12D3^{97Ile} protein having the SEQ ID NO: 4,
- administering substances, which are to be screened, to a subset of the subjects,
- comparing the predilection for ingredients of tobacco or tobacco smoke in subjects to which substances have been administered, with the predilection for ingredients of tobacco or tobacco smoke in subjects to which no substances have been administered, and
- detecting the specific binding of substances to the OR12D3^{97Ile} protein.

10. A method for diagnosing a predisposition to smoking comprising the steps of:
- providing at least one substance being specific to the OR12D3^{97Ile} protein having the SEQ ID NO: 4 in a vial and at least one substance that does not interact with the OR12D3^{97Ile} protein in another vial,
- subjecting a human to the vials sequentially,
- recording the olfactory recognition of the substances, and
- analyzing the records, wherein the recognition of substances being specific to the OR12D3^{97Ile} protein stands for a predisposition to smoking.


SEQUENCE LISTING

<110> Professor Dr. Andreas Ziegler

<120> Method for in-vitro diagnosing a predisposition to
      smoking and a pharmaceutical composition for treating
      tobacco abuse

<130> P502007EP-Va

<140> new
<141> 2007-08-27

<160> 4

<170> PatentIn Ver. 2.1

<210> 1
<211> 22
<212> DNA
<213> Artificial Sequence

<220>
<223> Description of Artificial Sequence: primer

<400> 1
agcgaagagg attgcagatg gc                                          22


<210> 2
<211> 32
<212> DNA
<213> Artificial Sequence

<220>
<223> Description of Artificial Sequence: primer

<400> 2
atacagccta tatcttttct aggctgtatc ac                               32


<210> 3
<211> 32
<212> DNA
<213> Artificial Sequence

<220>
<223> Description of Artificial Sequence: primer

<400> 3
atacagccta tatcttttct aggctgtatc at                               32


<210> 4
<211> 316
<212> PRT
<213> Artificial Sequence

<220>
<223> Description of Artificial Sequence: OR12D3^97Ile
      protein

<400> 4
Met Glu Asn Val Thr Thr Met Asn Glu Phe Leu Leu Leu Gly Leu Thr
  1               5                  10                  15

Gly Val Gln Glu Leu Gln Pro Phe Phe Phe Gly Ile Phe Leu Ile Ile
            20                  25                  30

Tyr Leu Ile Asn Leu Ile Gly Asn Gly Ser Ile Leu Val Met Val Val
        35                  40                  45

Leu Glu Pro Gln Leu His Ser Pro Met Tyr Phe Phe Leu Gly Asn Leu
    50                  55                  60

Ser Cys Leu Asp Ile Ser Tyr Ser Ser Val Thr Leu Pro Lys Leu Leu
65                  70                  75                  80

Val Asn Leu Val Cys Ser Arg Arg Ala Ile Ser Phe Leu Gly Cys Ile
                85                  90                  95

Ile Gln Leu His Phe Phe His Phe Leu Gly Ser Thr Glu Ala Ile Leu
            100                 105                 110

Leu Ala Ile Met Ala Phe Asp Arg Phe Val Ala Ile Cys Asn Pro Leu
        115                 120                 125

Arg Tyr Thr Val Ile Met Asn Pro Gln Val Cys Ile Leu Leu Ala Ala
    130                 135                 140

Ala Ala Trp Leu Ile Ser Phe Phe Tyr Ala Leu Met His Ser Val Met
145                 150                 155                 160

Thr Ala His Leu Ser Phe Cys Gly Ser Gln Lys Leu Asn His Phe Phe
                165                 170                 175

Tyr Asp Val Lys Pro Leu Leu Glu Leu Ala Cys Ser Asp Thr Leu Leu
            180                 185                 190

Asn Gln Trp Leu Leu Ser Ile Val Thr Gly Ser Ile Ser Met Gly Ala
        195                 200                 205

Phe Phe Leu Thr Leu Leu Ser Cys Phe Tyr Val Ile Gly Phe Leu Leu
    210                 215                 220

Phe Lys Asn Arg Ser Cys Arg Ile Leu His Lys Ala Leu Ser Thr Cys
225                 230                 235                 240

Ala Ser His Phe Met Val Val Cys Leu Phe Tyr Gly Pro Val Gly Phe
                245                 250                 255

Thr Tyr Ile Arg Pro Ala Ser Ala Thr Ser Met Ile Gln Asp Arg Ile
            260                 265                 270

Met Ala Ile Met Tyr Ser Ala Val Thr Pro Val Leu Asn Pro Leu Ile
        275                 280                 285

Tyr Thr Leu Arg Asn Lys Glu Val Met Met Ala Leu Lys Lys Ile Phe
    290                 295                 300

Gly Arg Lys Leu Phe Lys Asp Trp Gln Gln His His
305                 310                 315